# EUROPEAN PATENT APPLICATION

(11) **EP 4 134 439 A1**
(43) Date of publication of application: **15.02.2023**
(21) Application number: 21190764.7
(22) Date of filing: 11.08.2021
(51) Int. Cl.: C12N 15/82, A01N 63/60, C05B 7/00

(54) **PLANT EXTRACT AND USES THEREOF IN AGRICULTURE**

(71) Applicant: Valagro S.p.A., 66041 Atessa (Chieti) (IT)
(72) Inventor: Santaniello, Antonietta, 66041 ATESSA (IT); Perata, Pierdomenico, 56010 SAN GIULIANO TERME (IT); Povero, Giovanni, 66041 ATESSA (IT); Errico, Michela, 66041 ATESSA (IT); Biasone, Alessandro, 66041 ATESSA (IT); Piaggesi, Alberto, 66041 ATESSA (IT); Warrior, Prem, 66041 ATESSA (IT)
(74) Representative: Biggi, Cristina

(57) **Abstract**

The present invention refers to a plant extract, a formulation coprising said plant extract and uses thereof in agriculture, preferably for improving an agronomic performance of a receiving/target crop plant, such as corn, soybean, wheat, rice, tomato, melon, lettuce, and strawberry.

## Description

### TECHNICAL FIELD

The present invention refers to a plant extract, a formulation coprising said plant extract and uses thereof in agriculture, preferably for improving an agronomic performance of a receiving/target crop plant, such as corn, soybean, wheat, rice, tomato, melon, lettuce, and strawberry.

### BACKGROUND ART

The world's population is expected to grow to almost 10 billion by 2050 boosting the agricultural demand compared to the past. Therefore, a key question is whether today's agriculture and food systems can fulfill the needs of an extremely growing global population.

The consensus view is that current systems are likely capable of producing enough food, but to do so in an inclusive and sustainable manner will require important transformations.

Increased use of land, irrigation and agrochemicals played huge role in the growth of agricultural production during the Green Revolution. However, it is now recognized that the gains were often accompanied by negative effects on agriculture's natural resources, including land degradation, salinization of irrigated areas, over-extraction of groundwater, the build-up of pest resistance and the erosion of biodiversity. Agriculture has also damaged the wider environment through deforestation, the emission of greenhouse gases and nitrate pollution of water bodies.

To meet future food needs in a sustainable manner, agriculture has to keep surging with innovative systems and 'holistic' approaches aimed at protecting and enhancing the natural resources, while increasing productivity.

The technical solution herewith disclosed meets the needs reported above by providing agricultural biologicals which are both sustainable products and show remarkable benefits toward crop productivity. Therefore, the solutions here disclosed is a "green" approach to the challenge of producing enough crops to keep up with an increasing demand for food and fuel. The solutions proposed are based on natural or non-artificial small RNA molecules which are collected from donor plants and used to treat further receiving plants for the purpose of improving their agronomic traits of interest influenced by said small RNA molecules.

Applicant opened this innovative and unconventional agronomic approach with patent application WO2016/020874 wherein plant extracts or exudates comprising specific miRNAs naturally produced by plants were proposed for improving specific crop performances.

When it comes to the small RNA molecules approach in the agriculture sector, the "green" agronomic approach here proposed is distinct from the major trend. Indeed, most of the small RNA-based approaches known in the art rely on synthetic and/or GMO systems eventually leading to the production of the so-called small interfering RNAs (siRNAs) which regulate the expression of targeted genes through a RNA interference (RNAi) mechanism.

Applicant's approach is instead based on a different class of small RNA molecules: microRNAs (miRNAs), which play their biological effects by a similar mechanism even if siRNAs and miRNAs are substantially different from several angles as one of ordinary skill in the art would acknowledge.

In the Applicant's approach, miRNAs are gene-encoded in wild-type plants and are processed within donor plants before being collected (extracted) and applied to receiving plants; the artificial, dsRNA approach, instead, is based on spraying exogenously artificially designed dsRNA sequences, either chemically synthesized or produced utilizing a bacterial expression system, which - if taken up by the receiving plants - are then processed into the cell nucleus/citosol by the RNAi machinery to generate siRNAs. The results of Applicant approach were as unexpected as tremendously beneficial considering the above-mentioned landscape of a growing need for more food in a sustainable way. Moreover, the safety profile of a technology based on the utilization of small RNAs and miRNAs extracted from donor plants when compared to artificial dsRNA sequences with unpredictable effects on the consumer's health is of great relevance. The great advantages of the proposed solutions become even more evident along with the detailed disclosure and the non-limitative examples reported as follows.

### SUMMARY OF THE INVENTION

A first aspect of the present invention refers to a plant extract comprising: (a) up to 21% of small RNAs; (b) up to 16% of carbohydrates; (c) up to 36% of inorganic matter; and (d) the remainder, up to 76%, of small molecules and/or natural metabolites and/or relative by products.

According to a preferred embodiment, the extract comprises: (a) from about 5 to about 20% of small RNAs; (b) from about 1 to about 15% of carbohydrates; (c) from about 5 to about 35% of inorganic matter; and (d) from about 45 to about 75% of small molecules and/or natural metabolites and/or relative by products.

According to a preferred embodiment, the extract comprises: (a) from about 5 to about 10% of the small RNAs; (b) from about 1 to about 10% of the carbohydrate; (c) from about 10 to about 30% of inorganic matter; and (d) from about 50 to about 70% of small molecules and/or natural metabolites and/or relative by products.

Preferably the small RNAs are less than 200 nucleotides in length, preferably 20-25 nucleotides, more preferably 21-24 not or 18-24 nt or 19-22 nt or 21-22 nt. Preferably said one or more small RNAsis selected from: miR4995, miR159, preferably miR159a-3p and/or miR159e-3p, miR4371, preferably miR4371b, and combinations thereof. More preferably said one or more small RNAs define a profile of molecules selected from: miR4995 as the top expressed miRNA; and/or miR4995 and miR159 as the top two expressed miRNAs; and/or miR4995, miR159 and miR4371 or miR4995, miR159 and miR4371b or miR4995, miR159a-3p and miR159e-3p as the top three expressed miRNAs; and/or miR4995, miR159a-3p, miR159e-3p and miR4371b as the top four expressed miRNAs.

According to a preferred embodiment, the profile further comprise miR482, preferably miR482-5p and wherein the ratio between the relative expression level of miR4995 and miR482-5p (miR4995/miR482-5p) is greater or equal to 0.5, preferably greater or equal to 1, more preferably greater or equal to 50, still preferably greater or equal to 100.

According to a preferred embodiment, the extract further comprises one or more of or each of: peptides, proteins, free amino acids, oligosaccharides, natural chelating agents, cofactors, non-RNA nucleobase, saponins, phenols, and combinations thereof.

A further aspect of the present invention refers to a formulation comprising: (a) an extract according to any one of claims 1-8; and (b) an agricultural co-formulant and/or adjuvant. Preferably the agricultural formulant and/or adjuvant comprises stickers, fillers, binders, surfactants, dispersant, polymers, carriers, pH regulators, UV-protectants, and combinations thereof. Preferably the adjuvant reduces surface tension and/or improves solubilization, spreading, retention, and penetration of small RNAs.

A further aspect of the present invention refers to the extract or the formulation herein for use in agriculture, preferably for improving the agronomic performance or trait of a target crop plant. Preferably the extract or the is applied to the receiving crop plant at a concentration lower than or equal to 50 g/ha, preferably lower than or equal to 5g/ha, more preferably lower than or equal to 0.5 g/ha, still more preferably lower than or equal to 0.05 g/h wherein the concentration refers to the amount of small RNAs in grams per hectare, or applied to the receiving crop plant of step (i) at a concentration lower than or equal to 100 mg/L, preferably lower than or equal to 10 mg/L, more preferably lower than or equal to 1 mg/ha, still more preferably lower than or equal to 0.1 mg/ha wherein the concentration refers to the amount of small RNAs in milligrams per Liter.

Preferably the receiving crop plant is a monocotyledonous and/or dicotyledonous, preferably selected from: Grain crops, preferably cereals or pseudo cereals and legumes or pulses; the cereals are preferably selected from: maize (corn), millet, pearl or proso millet, sorghum, spring wheat or cool-season cereals, preferably selected from: barley, rye, rice, oats, spelt, teff, triticale, wheat and combinations thereof; the pseudo-cereals are preferably selected from: buckwheat, starchy grains from broadleaf, amaranth, buckwheat, chia, quinoa, and combinations thereof; other row crops, preferably selected from: cotton, sunflower, tobacco, canola, sugar cane, hop, peanut and combinations thereof; fruit and vegetable crops, preferably sugar beets, potatoes cabbages, cauliflower, broccoli, radish tomatoes, chicory, processing tomatoes, fresh tomatoes, eggplant, pepper, pumpkin, bell pepper, cucumber, zucchini, onion, garlic, lettuce, leafy vegetable, ginger, alkekengi, tea plant, celery, spinach, asparagus, fennel, berries in general and preferably strawberry, black berries, blue berries, peach, nectarines, apricot, cherry, plum, cedar, alchechengi, apple, pear, citrus, orange, tangerine, mandarin, clementin, satsuma, lemon, lime, pear, melon, water melon, cucumbers, onion, shallot, artichoke, lettuce, spinach, beets, carrots, table grape, wine grape, olive, kiwifruit, almond, walnut, hazelnut, pomegranate, and a combinations thereof; tropical crop preferably selected from banana, cocoa, mango, pineapple, coffee, cassava, coconut, papaya, passion fruit, guava, mangosteen, oil palm fruit, and a combinations thereof; forage crops, preferably selected from silage corn, Brachiaria, bermudagrass, festuca, Lolium, Poa, alfalfa, Trifolium spp., Vicia spp., forage pea, Sorghum silage, oats, millet, silage root crops, feed cabbage, clovers, rye, ryegrass, and combinations thereof; ornamental plants preferably selected from Roses, Ornamental bulb plants, Geranium, Begonia, Petunia, Spathiphyllum, Marantha, Chrysanthemum, Tulips, Sterlitzia, passionflower, hydrangea, orchids, spider plant, snake plants, lilac, marigold, rosemary, wisteria, aromatic plants, and combinations thereof.

Preferably the agronomic performance or trait is selected from: abiotic stress resistance and/or tolerance, preferably low or high temperature, deficient or excessive water, high salinity, heavy metals, and ultraviolet radiation; and/or nutrient use efficiency (NUE) and/or nutrient uptake, preferably said nutrient being selected from macro and/or meso/micro-nutrients, more preferably said macronutrients are selected from: nitrogen, phosphorus, potassium; and meso/micro-nutrients selected from: copper, sulphur, calcium, magnesium, iron, manganese, zinc, boron, and combinations thereof; and/or root growth and development and/or root biomass/weight and/or root system architecture; and/or shoot/canopy and/or root growth and development, biomass/weight; shoot greening, and/or yield potential and/or productivity of plants/crops; and/or plant fresh biomass and/or height and/or nutrient content and/or germination; and/or grain and/or fruit quality and quantity, number of pods, grain per pods, kernel line, grain weight, number of ears, flowering time, homogeneous budbreak/flowering, grain protein content, oil content, gluten strength, kernel size and vitreousness, fruit size, fruit setting, fruit color, fruit ripening, sugar content, improving flowering, pollination, and combinations thereof.

### BRIEF DESCRIPTION OF THE FIGURES

Fig. 1 shows results of the *in vitro* Vertical Plate experiments performed with different dosages of the small RNAs extract of the invention; in particular, the results are here reported in terms of exemplificative plant biology parameters such as root length (A-B) and biomass (C).
Fig. 2 shows the qualitative profile of the small RNAs extract - raw, not purified (A) and purified (B) - assessed using the "Bioanalyzer" (Agilent) to confirm the expected small RNA-related electropherogram peak.
Fig. 3 shows the results of *in vitro* Vertical Plate experiment in terms of root length performed with small RNAs extract (raw - total) and with the small RNAs component of the extract purified (A) or after the full extract is subject to RNase treatment for RNA component degradation (B).
Fig.4 shows the results of the "Seed Germination Pouch" screening system as shoot length (A) and shoot weight (B) after treatment with small RNAs extract (Raw/Total) and with the small RNA component purified.
Fig. 5 shows the phenomic results obtained by testing the small RNAs extract at different concentrations in terms of Digital Biomass (A-B) and Dark Green Color (C) compared to internal positive control.
Fig. 6 shows the correlation measure in field trials on corn between Nitrogen content in the leaves and the final yield of the crop after treatment with the small RNA extract of the invention.

### DETAILED DESCRIPTION OF THE INVENTION

Unless defined otherwise, all technical and scientific terms used herein have the same meaning as commonly understood by one of ordinary skill in the art to which this invention belongs. Where there are discrepancies in terms and definitions, the definitions given herein must be considered as prevailing. Other technical terms used herein have their ordinary meaning in the art in which they are used, as exemplified by various art-specific dictionaries.

A first aspect of the present invention refers to a method for improving the agronomic performance or at least one agronomical trait in a target plant, said method comprising the following steps:
(i) providing a plant to be treated, said plant being a target plant;
(ii) Extracting one or more small RNA molecules (herein also named small RNAs or sRNAs) from a donor plant thereby obtaining an extract of a donor plant comprising one or more small RNAs;
(iii) Applying the extract of step (ii) to the target plant of step (i) at a dosage or application rate lower than or equal to 50g/ha, preferably lower than or equal to 5g/ha, more preferably lower than or equal to 0.5 g/ha, even more preferably lower than or equal to 0.05 g/ha, wherein g/ha means grams of small RNAs per hectare.

Alternatively, the application rate can be also expressed as lower than or equal to 100 mg/L, preferably lower than or equal to 10 mg/L, more preferably lower than or equal to 1 mg/L, even more preferably lower than or equal to 0.1 mg/L, wherein mg/L means milligrams of small RNAs per Liter of solution applied onto the receiving plant.

Step (ii) may be also disclosed as follows: obtaining an extract from a donor plant or part thereof, said extract comprising one or more small RNAs.

In other words, the invention refers to a method to improve one or more agronomic traits of interest in a target plant, wherein the target plant is preferably a commercially relevant crop. Said improvement is the consequence of the application of the extract from the donor plant comprising one or more small RNAs at specific dosages or application rates. The small RNAs contained into the extracts are the Active Ingredient (Al), named also active constituent/substance/principle; therefore, the small RNAs as Al are responsible for the biological activities or phenotypic evidence or the one or more agronomic traits triggered into the receiving plant following application of the extract from the donor plant comprising small RNAs.

In this context, "a plant extract comprising small RNAs" and "a plant extract comprising one or more of small RNAs" are synonyms and mean: an extract obtained from a donor plant as defined herein, the extract comprising a concentrated amount of small RNAs endogenously produced by the donor plant. The one or more small RNAs may be a plurality of small RNAs. For simplicity, from now on said "plant extract comprising small RNAs" or said "plant extract comprising one or more of small RNAs" as defined above will be mentioned as "extract" or "small RNAs extract".

In this context, "method" also means a process, a use or an application protocol, preferably an agriculture application or an agriculture practice.

In this context, "donor plant" refers to a plant or a plurality of plants or any part thereof from which the small RNAs are extracted; therefore, the donor plant(s) is/are the source of the extract comprising the small RNAs as defined herein. The small RNAs are preferably endogenously and/or naturally produced by the donor plant; therefore, the donor plant is preferably not genetically modified to produce said small RNAs and is non-GM and/or an edited plant. The small RNAs are extracted from the donor plant, and subsequently fed to a receiving plant wherein they exercise their benefit in terms of biological effects or agronomic performance.

In this context, the "target plant" or "target plants" can be also named "receiving or treated plant(s)". The target plant is distinct from the donor plant and is the plant treated with the method here disclosed for the purpose of improving at least one agronomical trait of interest. The target plant is the plant benefiting from the method of the disclosure and may belong to the same species of the donor plant and/or to a species which is different from that of the donor plant.

Indeed, the Applicant has surprisingly discovered that the agronomic performance of plants treated with the small RNAs extract herein can be modulated or altered by the dosage or application rate of said small RNAs. In particular, the Applicant has demonstrated the drop or loss of the biological effects or the drop or loss of the improvements of one or more agronomic traits in a receiving plant when said receiving plant is treated at a dosage (application rate) above 50g/ha or 100 mg/L wherein the dosage refers to the amount of small RNA applied per hectare. Indeed, as reported in the exemplificative and not limitative embodiments herein described, Applicant has found out that when a receiving plant, preferably a crop, is treated with a dosage or application rate lower than or equal to 50g/ha or 100 mg/L (the dosage refers to the amount of small RNA applied per hectare) the receiving plant shows enhancement of one or more, preferably a plurality, of agronomic traits of interest. The one or more agronomic traits that are enhanced are, preferably selected from: root growth, root mass, increase of yield, fresh biomass, greening, better use of nutrients and combinations thereof.

According to a preferred embodiment, the best performance in terms of the enhancement or amelioration of the one or more agronomic traits of interest is obtained when the extract is applied to a target plant at a dosage or an application rate lower than or equal to 5g/ha or 10 mg/L, preferably lower than or equal to 0.5 g/ha or 1 mg/L, more preferably lower than or equal to 50 mg/ha or 0,1 mg/L (the dosage refers to the amount of small RNA applied per hectare). In other words, Applicant has unexpectedly demonstrated that the dosage or application rate of the small RNAs comprised in an extract obtained from a donor plant, wherein the donor plant naturally produces said small RNAs, plays a critical role in triggering the molecular mechanisms which lead to the improvement of the one or more agronomic traits of interest.

In this context, "agricultural biologicals" or simply "biologicals" are a diverse group of products derived from naturally occurring microorganisms, plant extracts and plant-derived materials, beneficial insects, or other organic matter to be used in agriculture preferably for plant/crop biostimulation or enhancement, the so-called Plant Biostimulants (PBs) and/or for plant protection, the so-called biopesticides or biocontrol products (PPPs).

In this context, "Plant Biostimulants" or even "biofertility products" are also named plant growth/productivity enhancement products or plant nutrition products. The PBs have a dynamic definition framework - especially from regulatory perspective; indeed, so far, the most accredited definition in Europe is the following: "a product stimulating plant nutrition processes independently of the product's nutrient content with the sole aim of improving one or more of the following characteristics of the plant or the plant rhizosphere: (a) nutrient use efficiency, (b) tolerance to abiotic stress, (c) quality traits, (d) availability of confined nutrients in soil or rhizosphere". In the U.S. instead, a Plant Biostimulant means a "substance or micro-organism that, when applied to seeds, plants, or the rhizosphere, stimulates natural processes to enhance or benefit nutrient uptake, nutrient efficiency, tolerance to abiotic stress, crop quality or yield". However, as mentioned above, the Plant Biostimulant is a very fluid and transforming agronomic field.

According to a preferred embodiment, the receiving plant is any plant species belonging to the angiosperm division and therefore either monocotyledonous and/or dicotyledonous. Preferably said receiving plant is a crop plant species selected from:
- Grain crops, preferably cereals or pseudo cereals and legumes or pulses; the cereals are preferably selected from: maize (corn), millet, pearl or proso millet, sorghum, spring wheat or cool-season cereals, preferably selected from: barley, rye, rice, oats, spelt, teff, triticale, wheat; the pseudo-cereals are preferably selected from: buckwheat, starchy grains from broadleaf, amaranth, buckwheat, chia, quinoa;
   said legumes or pulses are selected from: chickpeas, common beans, common peas (garden peas), fava beans, lentils, lima beans, lupins, mung beans, peanuts, pigeon peas, runner beans, soybeans and combination thereof;
- other row crops, preferably selected from: cotton, sunflower, tobacco, canola, sugar cane, hop, peanut and combination thereof;
- fruit and vegetable crops, preferably sugar beets, potatoes cabbages, cauliflower, broccoli, radish tomatoes, chicory, processing tomatoes, fresh tomatoes, eggplant, pepper, pumpkin, bell pepper, cucumber, zucchini, onion, garlic, lettuce, leafy vegetable, ginger, alkekengi, tea plant, celery, spinach, asparagus, fennel, berries in general and preferably strawberry, black berries, blue berries, peach, nectarines, apricot, cherry, plum, cedar, alchechengi, apple, pear, citrus, orange, tangerine, mandarin, clementin, satsuma, lemon, lime, pear, melon, water melon, cucumbers, onion, shallot, artichoke, lettuce, spinach, beets, carrots, table grape, wine grape, olive, kiwifruit, almond, walnut, hazelnut, pomegranate, and a combination thereof;
- tropical crop preferably selected from banana, cocoa, mango, pineapple, coffee, cassava, coconut, papaya, passion fruit, guava, mangosteen, oil palm fruit, and a combination thereof;
- forage crops, preferably selected from silage corn, Brachiaria, bermudagrass, festuca, Lolium, Poa, alfafa, Trifolium spp., Vicia spp., forage pea, Sorghum silage, oats, millet, silage root crops, feed cabbage, clovers, rye, ryegrass, and combination thereof;
- ornamental plants preferably selected from Roses, Ornamental bulb plants, Geranium, Begonia, Petunia, Spathiphyllum, Marantha, Chrysanthemum, Tulips, Sterlitzia, passionflower, hydrangea, orchids, spider plant, snake plants, lilac, marigold, rosemary, wisteria, aromatic plants, and combination thereof.

The most preferred receiving plants are selected from row crops; more preferably the receiving plants are selected from: corn, soybean, rice, wheat, canola and combination thereof, and/or from fruits and vegetables crops, preferably from lettuce, tomato, melon, strawberry, and combination thereof.

In this context "crop" means a plant or animal product that can be grown and harvested extensively for profit or subsistence. Crops may refer either to the harvested parts or to the harvest in a more refined state. Most crops are cultivated in agriculture or aquaculture. A crop may include macroscopic fungus (e.g., mushrooms), or alga. Most crops are harvested as food for humans or fodder for livestock. Some crops are gathered from the wild (including intensive gathering, e.g., ginseng).

According to a preferred embodiment of the invention, the donor plant from which the small RNAs are extracted is the whole plant and/or any part thereof. Preferably said any part is selected from: leaves, seeds, roots, seedlings, stems, flowers, tubers, bulbs, rhizomes, fruits, branches, and part thereof such as peels, fruit skin and combination thereof. The part of plants may also come from the production or processing, post-processing of said donor plants, and therefore may be by-products or waste or secondary products of said donor plants.

The donor plant is any mono or dicotyledon plant. Preferably the donor plant is selected from legumes. In this context, "Legumes" mean a plant belonging to the family Fabaceae or Leguminosae, preferably selected from: clover, mesquite, fava bean, amarind, alfalfa, broad bean, read bean, black bean, carob, chickpea, cowpea, fenugreek, green bean, lentil, licorice, lima, bean pea, peanut, scarlet runner bean, soybean, tamarind, forage and fodder, alfalfa bird's-foot, trefoil bush clover, hyacinth bean, lupine, silk tree sun hemp, acacia, and combination thereof.

In this context, "agronomic trait" means any plant trait or plant distinguishing parameter or even plant phenotype having agronomic relevance.

Preferably the agronomic trait is selected from:
- abiotic stress resistance and/or tolerance, preferably low or high temperature (chilling or heat stress), deficient or excessive water (water scarcity/drought or flooding), high salinity, heavy metals, and ultraviolet radiation; and/or
- nutrient use efficiency (NUE) and/or nutrient uptake, preferably said nutrient being selected from macro and/or meso/micro-nutrients, more preferably said macronutrients are selected from: nitrogen (N - and so Nitrogen Use Efficiency), phosphorus (P- and so Phosphorous Use Efficiency), potassium (K); and meso/micro-nutrients selected from: copper (Cu), sulphur (S), calcium (Ca), magnesium (Mg), iron (Fe), manganese (Mn), zinc (Zn), boron (B),and combination thereof; and/or
- root growth and development and/or root biomass/weight and/or root system architecture; and/or
- shoot/canopy and/or root growth and development, biomass/weight; shoot greening, and/or
- yield potential and/or productivity of plants/crops; and/or
- plant fresh biomass and/or height and/or nutrient content and/or germination; and/or
- grain and/or fruit quality and quantity, number of pods, grain per pods, kernel line, grain weight (cereal and Leguminosae), number of ears (corn), flowering time, homogeneous budbreak/flowering, grain protein content (GPC), oil content, gluten strength, kernel size and vitreousness, fruit size, fruit setting, fruit color, fruit ripening, sugar content, improving flowering, pollination, and combination thereof.

According to the most preferred embodiment, the agronomic trait of interest is Nutrient Use Efficiency or NUE, nutrient uptake, yield potential, yield productivity, preferably crop productivity and combinations thereof.

In this context, "Nutrient Use Efficiency" refers to the plant's ability to take up nutrients efficiently from the soil or a better use of them, and/or plant's ability of internal transport, storage, and remobilization of nutrients. Preferably the NUE improvement in the target plants is shown when said target plant is under optimal or balanced plant nutrition or under suboptimal conditions of nutrients or inorganic fertilizer.

In this context, "optimal or balanced plant nutrition" means the application to the plants/crops of an integrated approach providing the standard or adequate (100%) nutritional needs of a specific crop throughout its life cycle, considering - *inter alia* - the removal of nutrients, agro-techniques, agro-climatic situations, soil chemical and physical characteristics. An optimal or balanced plant nutrition ensures supplying fertilizers in optimum quantities and proper proportion through the most suitable methods, resulting in the sustenance of soil fertility, crop yield/productivity, and quality.

In this context, "suboptimal plant nutrition" means providing the nutritional needs of a specific crop in sub-optimum/reduced quantities (from -10% to -50%) compared to the optimal or balanced plant nutrition condition (that is 100%). Or nutrient availability below the optimum level, despite supplying fertilizers in optimum amounts and proper proportion, due to limiting environmental factors (soil properties, rainfall, *etc*.).

According to a preferred embodiment, the application of the small RNAs extract to the receiving plants at the dosage reported above allows receiving plants to improve or make efficient the nutrient use, in particular Nitrogen Use, preferably in combination with yield increase. In other words, when applied to the receiving plant, such as corn or soybean, the small RNAs extract significant improves the efficient Nutrient Use, in particular with reference to one or more of the following nutrients: Nitrogen, Phosphorous, Potassium, Boron, Calcium, Manganese, Iron, Copper or Potassium, and preferably also a significant increase in the final yield of the crop/plant. This is the most desirable and main achievement for any grower. Indeed, what matters for growers is the final yield and it is very desirable for growers and for the environment when a solution, like the one herein disclosed, can assure good or even improved yield of the plant(crop) by using few nutrients or by stimulating crops at using the nutrients in a better or more efficient way, in particular key nutrients such as Nitrogen or Phosphorous or Potassium. Reducing fertilizers rate applications is a very sensitive topic at all levels especially for climate change; indeed, a global, huge and common effort is tremendously requested toward this direction and this solution becomes even ideal when the goal is achieved by improving also crop performance.

The extraction step is performed on a donor plant since, as mentioned above, the small RNA molecules are extracted from a donor plant and then applied to a receiving plant. Therefore, the receiving plant is treated with naturally derived and exogenous small RNA molecules since these small RNAs are produced naturally by a plant (i.e. the donor plant) that is distinct from the receiving plant. In other words, the small RNAs are produced endogenously by the donor plant (therefore externally from the receiving plant) and then the small RNAs extracted from the donor plant are applied to the receiving plant (exogenous application). However, said receiving plant expresses their own small RNAs which may in some cases also correspond to some or all of the small RNA molecules extracted from the donor plant, even though the small RNA concentration in the receiving plants can be different from that achieved by exogenously feeding them with the small RNAs extract derived from the donor plant. Because the receiving plant is different from the donor plant, in some embodiments the receiving plant may receive an extract with a small RNA composition (plurality/panel) different from the small RNAs endogenously produced by the receiving plant, thereby providing the receiving plant with one or more new small RNAs as bioactive molecules.

As said, the small RNA molecules are naturally produced by the donor plant that is preferably wild type, therefore the donor plant is preferably not genetically engineered or a transgenic plant (non-Genetically Modified Organism - GMO). In any case, the small RNA molecules present in the extract are endogenously and/or naturally produced by the donor plant and therefore the small RNAs are not artificially designed/unnatural or synthetic/man-made by chemical synthesis or expressed by bacterial systems.

In this regard, it should be noted that the small RNAs, like other biological molecules, undergo post-transcriptional modifications, such as methylation, that are key for gene expression modulation in eukaryotes. Just to provide some examples, it is known that plant miRNAs possess a 2'-*O*-methyl group on the ribose of the 3' terminal nucleotide, and that this methyl group is added after miRNA/miRNA*formation to protect miRNAs from 3' terminal uridylation and subsequent degradation (https://www.ncbi.nlm.nih.qov/pmc/articles/PMC5134323/). Moreover, scientific literature reporting miRNA methylation at adenosine, guanosine and cytosine has been increasing recently, in particular, with reference to cancer and related prognosis (https://molecular-cancer.biomedcentral.com/articles/10.1186/s12943-020-01155-z). Growing evidence indicates that post-transcriptional modifications of molecules and in general epigenetic, are becoming crucial in influencing cell biology and consequently the phenotypes and traits of organisms. From this angle, the post-transcriptional modification should be considered a relevant point of differentiation between artificial/synthetic or even prokaryotic produced small RNAs and naturally produced small RNAs since only naturally produced molecules may guarantee 100% correct post-transcriptional modifications and epigenetic inheritance.

In this context, "wild type" (WT) refers to the phenotype of the typical form of a species whose genotype is as it occurs in nature or to cultivars that do not occur in nature; these cultivars are non-GM plants and/or non gene-edited. Herein "transgenic" refers to such plants that have been genetically engineered by using recombinant DNA techniques to create plants with new characteristics. Transgenic plants are identified as a class of genetically modified organisms (GMO).

In this context, "small RNA molecules" (sRNAs or small RNAs) are non-coding RNAs (ncRNAs) that generally do not encode proteins and are composed of less than 200 nucleotides (nt); however, such RNAs contain biological information controlling various levels of gene expression, including chromatin architecture, epigenetics, transcription, splicing, editing, mRNA stability, and mRNA translation. More and more evidence indicate that the majority of mammalian and other complex organism genomes are transcribed into ncRNAs. Among small RNAs it is possible to distinguish microRNAs (miRNAs) and short interfering RNAs (siRNAs). Mature miRNAs and siRNAs are about 18-22 nt and 20-24 nt long, respectively, that regulate gene expression, usually, post-transcriptionally by base-pairing to complementary transcripts. Therefore, preferably, the small RNA molecules of the present invention are less than 200 nt length preferably 18-24 nt length, more preferably 20-22 nt length, still more preferably miRNAs having 20-24 nt, preferably miRNA of 21-22 nt length. In this context nucleotide (nt) may be also base pair (bp).

Preferably said small RNAs, preferably said miRNA, are applied as naked molecules meaning that the molecules are not conjugated or flagged or bound to any other molecules following human or lab or artificial intervention.

In plants, miRNAs are processed from longer hairpin-shaped precursors encoded in the genome and almost all of them are transcribed by RNA polymerase II. This pri-miRNA (i.e. primary miRNA transcript) is first processed into a precursor, the pre-miRNA, and then excised as RNA duplex by the endonuclease enzyme Dicer-like 1 (DCL1). The resulting duplex sequence, composed by the guide miRNA and the complementary miRNA* (also known as passenger miRNA), is usually, but not always, destined for degradation. The guide miRNA, but possibly also the miRNA*, is then exported to the cytoplasm by diverse factors, including the HASTY protein. Current knowledge is lacking about the spatio-temporal separation of the two strands but once performed, the guide miRNA is loaded onto a member of the AGO protein family to assemble the RNA-induced silencing complex (RISC). The miRNA can then play its regulatory role by specifically binding a target transcript based on sequence complementarity. The regulatory processes performed by miRNAs are widely conserved in plants, animals, protists, and fungi, highlighting the significant influence that these regulators can have on the evolution of gene expression.

Plant miRNAs are involved in most, if not all, biological processes and have been found in all the organs where they have been searched for. These sequences are key regulators in important processes such as hormone regulation, nutrient homeostasis, development and interaction with pathogens and symbionts, as well as with environmental stresses. As part of some of these mechanisms and processes, miRNAs can act indirectly on gene regulation, triggering the production of other small RNAs, known as secondary siRNA. Some of these molecules are called phasiRNAs, for phased small interfering RNAs.

The small RNAs molecules of the invention are extracted from the donor plants or part thereof by using one or more processes known in the art. Any process allowing the extraction and/or the enrichment and/or the concentration of the small RNAs from plants (donor plants) naturally (endogenously) producing said small RNAs should be considered useful for the purpose of the present invention, and therefore herewith disclosed.

Examples of extraction processes used to extract the small RNAs according to the invention are:
- Organic Extraction Methods also known as Phenol-based RNA isolations that represent the gold standard for RNA preparation, or
- Filter-based or Column-based RNA isolation methods, such as Spectrum plant total RNA kit (Sigma Aldrich), RNeasy^{®}(Qiagen) and miRCURY (Exiquon), or
- Magnetic Particle Methods, such as MagMAX mirVana Total RNA (phenol-free-Thermo Fisher).

Alternatively, for this purpose plants or parts thereof may be treated with a basic solution, preferably a bicarbonate solution, at high temperature. The process is disclosed in more detail in the WO2017072285 herewith incorporated by reference. Preferably the donor plants or part thereof are treated with a bicarbonate solution, preferably an alkali metal bicarbonate solution, for at least a few hours, preferably from 1 to 24 hours, at a temperature ranging preferably from 50 to 100°C. After the extraction treatment, the liquid part contains the extract with the small RNAs component. The liquid part is preferably mixed with an alcohol, such as isopropanol or ethanol, to allow RNAs precipitation. Small RNAs extract may be collected by using any means known in the art for the purpose.

According to a preferred embodiment of the invention, the extract comprises small RNAs, preferably concentrated amount of small RNAs endogenously produced by a donor plant at a concentration ranging from 1 to 20%, in other words the extract is characterized by a titration (title) of small RNAs endogenously produced by a donor plant ranging from 1 to 20%, preferably from 5 to 10% wherein said small RNAs are preferably characterized by a length <200 ribonucleotides (nt) or base pair (bp), more preferably their size ranges between 21 and 24 nt or bp.

According to a preferred embodiment of the invention, besides the small RNA component disclosed above, the extract comprises carbohydrates and/or inorganic matter and/or small molecules and/or natural metabolites and/or relative by-products.

According to a preferred embodiment of the invention, besides the small RNA component disclosed above, the extract comprises carbohydrates wherein the carbohydrate component is present as average 1-15%.

According to a preferred embodiment of the invention, besides the small RNA component disclosed above, the extract comprises the inorganic matter wherein the inorganic matter is present as average 10-30%.

According to a preferred embodiment of the invention, besides the small RNA component disclosed above, the extract comprises small molecules and/or natural metabolites and/or relative by-products wherein they are present as average 25-40%.

According to a preferred embodiment of the invention, the extract comprises
- small RNAs endogenously produced by a donor plant ranging from 1 to 15%; and/or
- carbohydrates wherein the carbohydrate components are present as average 1-15; and/or
- inorganic matter wherein the inorganic matter is present as average 10-30%; and/or
- small molecules and/or natural metabolites and/or relative by-products as average 25-40%

The most homogeneous class of molecules of the extract is the small RNAs, while the remaining classes of molecules are heterogeneous and belong to the following families: peptides, free amino acids, oligosaccharides, natural chelating agents, cofactors, nucleobases, saponins and phenols.

A further embodiment of the invention refers to a plant extract comprising:
(i) an RNA component of any of the embodiments shown in Table A; and/or
(ii) a carbohydrate component of any of the embodiments shown in Table B; and/or
(iii) an inorganic matter component of any of the embodiments of any of the embodiments shown in Table C.; and/or
(iv) a small molecule and/or small molecules and/or natural metabolites and/or relative by-products component of any of the embodiments shown in Table D.

| **Table A - RNA component may be any combination of the amounts and lengths shown below:** | |
|---|---|
| **Amount of small RNA** | **Length of RNA** |
| from about 1 to about 20%; from about 1 to about 10%; from about 5 to about 15%; from about 3 to about 11%; or from about 5 to about 10%; or up to 21% | < 200 nucleotides; < 150 nucleotides; < 100 nucleotides; < 50 nucleotides; < 30 nucleotides; 20-30 nucleotides; 20-25 nucleotides; 21-24 nucleotides |

| **Table B - Carbohydrate component may be present in any of the amounts shown below:** |
|---|
| **Amount of Carbohydrate** |
| up to 16%; from about 1 to about 15%; from about 1 to about 20%; from about 1 to about 30%; from about 1 to about 16%; from about 1 to about 11%; from about 1 to about 12%; or from about 1 to about 13% |

| **Table C - The inorganic matter component may be present in any of the amounts shown below:** |
|---|
| **Amount of inorganic matter component** |
| < 35%; < 50%; from about 5 to about 35%; from about 10 to about 30%; from about 5 to about 30%; or up to 31% |

| **Table D - The small molecules and/or natural metabolites and/or relative by products may be present in any of the amounts shown below:** |
|---|
| < 80%; < 70%; < 60 %; < 65%; from about 45 to about 75%; from about 45 to about 70%; from about 50 to about 70%; from about 50 to about 65%; from about 50 to about 60%; from about 55 to about 70%; or from about 55 to about 65% |

The component for Tables C and D may be one or more of or each of: peptides, free amino acids, oligosaccharides, natural chelating agents, cofactors, non-RNA nucleobase, saponins, and/or phenols.

Therefore a further aspect of the present invention refers to a plant extract comprising: (a) up to 21% of small RNAs; (b) up to 16% of carbohydrates; (c) up to 36% of inorganic matter; and (d) the remainder, up to 76%, of small molecules and/or natural metabolites and/or relative by-products.

Preferably the plant extract comprises: (a) from about 5 to about 20% of small RNAs; (b) from about 1 to about 15% of carbohydrates; (c) from about 5 to about 35% of inorganic matter; and (d) from about 45 to about 75% of small molecules and/or natural metabolites and/or relative by-products.

More preferably, the extract comprises: (a) from about 5 to about 10% of the RNA; (b) from about 1 to about 10% of the carbohydrate; (c) from about 10 to about 30% of inorganic matter; and (d) from about 50 to about 70% of small molecules and/or natural metabolites and/or relative by-products.

The RNA is preferably less than 200 nucleotides in length and preferably is 20-25 nucleotides or 21-24 nucleotides in length.

The extract preferably further comprises one or more of or each of: peptides, free amino acids, oligosaccharides, natural chelating agents, cofactors, non-RNA nucleobase, saponins, phenols, and combinations thereof.

According to a preferred embodiment of the invention, the extract comprising/enriched/concentrated in small RNAs from a donor plant is used as such, that is alone, or alternatively formulated by using any co-formulant and/or adjuvant useful for agricultural purposes. The co-formulant and/or adjuvant is/are preferably selected from: stickers, fillers, binders, surfactants, dispersants, polymers, carriers, a pH regulators, UV-protectants, and combination thereof.

Several combinations of different kinds of adjuvants/co-formulant, preferably as secondary and/or ternary complex, may be used in the context of the present invention. The adjuvants are used preferably to reduce surface tension and/or to improve solubilization, spreading, retention and penetration of the small RNAs.

The sticker is preferably used - *inter alia* - for long-lasting attaching or residing of small RNAs on the plant/leaf surface; in other words, to increase the time of stationing of the extract on the plant/leaf surface. The sticker useful for the purpose of the present invention is preferably selected from: Xylitol, Sorbitol, Xanthan Gum, Polyquaternium 37, such as Atlox Rheostrux 300A, corn starch, maltodextrin, and glucose and combinations thereof.

The surfactant is preferably used to improve the penetration of the extract into the receiving plants. The surfactant useful for the purpose of the present invention is preferably selected from: Cocamidopropyl Betaine, Lauroyl Arginine, preferably Amisafe AL-01, Atplus^{™} DRT-NIS, preferably Non-ionic surfactant, PEG, preferably Kollisolv PEG 8000, alkyl polyethylene glycol ethers, preferably Lutensol ON and combinations thereof.

The polymer is preferably used to neutralize the charge of the small RNAs of the extract and to improve the transport and penetration of the extract. Said polymer is preferably a cationic polymer, preferably selected from Polyethylenimine (PEI), Poly(L-lysine) (PLL), Polyamidoamine (PAA), Poly(2-dimethylaminoethyl methacrylate) (PDMAEMA), Poly(beta-amino esters) (PAE), CD), Gelatine, Cellulose (CS), Hyaluronic Acid (HA), Epigallocatechin Gallate (EGCG), Pullulan (PU), and β-Cyclodextrin (β-CS) and combination thereof.

The filler is preferably selected from: sucrose, lactose, saccharose, maltodextrin and combinations thereof.

The binder is preferably selected from: starch, gluten, lecithin (natural or modified), polyethylene glycols and combinations thereof.

The dispersant is preferably selected from: lignosulfonates, sorbitan fatty acid esters, ethoxylated fatty amines, ethoxylated fatty esters, glycerol esters, monoglycerides, sulfates sucrose and glucose esters, and sulfonates of oils and fatty acids, monoglycerides, fatty acids, and combinations thereof.

The pH adjuster is preferably selected from: citric acid, tartaric acid, lactic acid, malic acid and combinations thereof.

Therefore a further aspect of the invention refers to a formulation comprising:
(a) a small RNA extract as disclosed herein; and
(b) an agricultural co-formulant and/or adjuvant.

Preferably the agricultural formulant and/or adjuvant comprises stickers, fillers, binders, surfactants, dispersants, polymers, carriers, pH regulators, UV-protectants, and a combination thereof. More preferably the adjuvant reduces surface tension and/or improves solubilization, spreading, retention, and penetration of small RNAs.

According to a preferred embodiment, the small RNAs extract is used in combination with at least one of the following ingredients: plant biostimulants (PBs), biopesticides (PPP), nitrogen sources, phosphorus sources, potassium sources, magnesium and/or calcium sources, sulfur sources, iron and/or manganese and/or zinc and/or copper sources, PGR, and combinations thereof.

Therefore, a further aspect of the present invention refers to a composition comprising the small RNAs extract as disclosed above and at least one of the following further ingredients: plant biostimulants (PBs), biopesticides (PPP), nitrogen sources, phosphorus sources, potassium sources, magnesium and/or calcium sources, sulfur sources, iron and/or manganese and/or zinc and/or copper sources, bacteria (microbials), fungi, yeasts, PGR, proteins, peptides, amino acids, the adjuvant/co-formulants as disclosed above and combinations thereof. Said composition is preferably for using in the method or agronomic practice disclosed herein.

Plant Biostimulant or PBs definition has been reported above and it preferably refers to an ingredient or a mixture or a composition or a product based on one or more of the following ingredients: plants and derivatives thereof, algae and derivatives thereof, microalgae, and derivatives thereof; optionally said composition may comprise also animal derivatives. As used herein, "derivatives" preferably refer to: extracts, exudates, lysates, hydrolysates, processing bioproducts, residues, wastes and combinations thereof. More preferably said derivatives are selected from: humic acids, fulvic acids, seaweed extracts, botanicals, chitosans, biopolymers and combinations thereof.

As used herein, "fungi" preferably refer to mycorrhizal and/or non-mycorrhizal fungi, and microbials are preferably bacterial endosymbionts, preferably belonging to the genus Rhizobium and/or Plant Growth-Promoting Rhizobacteria (PGPR).

As used herein, "algae" refer to a functional group of organisms that carry out oxygenic photosynthesis and are not embryophytes. They include both bacterial (cyanobacteria) and/or eukaryotic organisms. The term encompasses organisms that are photoautotrophic, heterotrophic, or mixotrophic, and are typically found in freshwater and marine systems. The term algae includes macroalgae and/or microalgae. Preferably, said macroalgae are seaweed, preferably red, brown or green, wherein said brown seaweed is selected from: *Ascophyllum nodosum, Ecklonia maxima, Laminaria saccharina, Laminaria digitata, Fucus spiralis, Fucus serratus, F. vesiculosus, Macrocystis spp., Pelvetia canaliculata, Himantalia elongata, Undaria pinnatifida,* Sargassum spp, and combinations thereof; wherein said red seaweed is selected from: Kappaphycus spp., Chondrus spp., Palmaria spp., Gracilaria spp., Porphyra spp., Porphyridium spp., Mastocarpus spp., Polysiphonia spp.; wherein said green seaweed is selected from: Ulva spp., Caulerpa spp., Codium spp., Halimeda spp, Acetabularia spp., Cladophora spp. *Ascophyllum nodosum* is particularly preferred for the purposes of the present invention.

As used herein, "microalgae" refer to any microscopic algae that are unicellular and simple multi-cellular microorganisms, including both prokaryotic microalgae, preferably, cyanobacteria (Chloroxybacteria), and eukaryotic microalgae, preferably green algae (Chlorophyta), red algae (Rhodophyta), or diatoms (Bacillariophyta). Preferably said microalgae are selected from: Spirulina, Scenedesmus, Nannochloropsis, Haematococcus, Chlorella, Phaeodactylum, Arthrospyra, Tetraselmis, Isochrysis, Synechocystis, Clamydomonas, Parietochloris, Desmodesmus, Neochloris, Dunaliella, Thalassiosira, Pavlova, Navicula, Chaetocerous, and combinations thereof.

As used herein, "plant" means any one of the vast numbers of organism within the biological kingdom Plantae. Conventionally the term plant implies a taxon with characteristics of multicellularity, cell structure with walls containing cellulose, and organisms capable of photosynthesis.

Modern classification schemes are driven by somewhat rigid categorizations inherent in DNA and common ancestry. Preferably, they include monocotyledonous and dicotyledonous species including trees, forbs, shrubs, grasses, vines, ferns, mosses, and crop plants, preferably vegetables, orchards, and row crops. Preferably said plant is selected from: sugar beet, sugar cane, corn, alfalfa, maize, brassica, halophytes, soya, wheat, yucca, quillaja, hop, coffee, citrus, olive, and combinations thereof.

As used herein "Plant Growth-Promoting Rhizobacteria (PGPR)" means a group of bacteria that enhances plant growth and yield via various plant growth promoting substances as well as biofertilizers. Preferably the PGPR is selected from: *Aeromonas rivuli, Agromyces fucosus, Bacillus spp. Bacillus mycoides, Bacillus licheniformis, Bacillus subtilis, Bacillus megaterium, Bacillus pumilus, Bacillus safensis, Microbacterium sp., Nocardia globerula, Stenotrophomonas spp., Pseudomonas spp, Pseudomonas fluorescens, Pseudomonas fulva, Pseudoxanthomonas dajeonensis, Rhodococcus coprophilus, Sphingopyxis macrogoltabida, Streptomyces spp., Enterobacter spp., Azotobacter spp., Azospiriullum spp., Rhizobium spp., Herbaspirillum spp., Lactobaccillus spp., Lactobacillus acidophilus, Lactobacillus buchneri, Lactobacillus delbrueckii, Lactobacillus johnsonii, Lactobacillus murinus, Lactobacillus paraplantarum, Lactobacillus pentosus, Lactobacillus plantarum, Lactococcus tactis,* and combinations thereof.

As used herein yeast is preferably selected from: *Candida spp., Candida tropicalis, Saccharomyces spp., Saccharomyces bayanus, Saccharomyces boulardii, Saccharomyces cerevisiae, Saccharomyces exiguous, Saccharomyces pastorianus, Saccharomyces pombe,* and combinations thereof; and/or

As used herein the mycorrhiza is preferably selected from: *Glomus spp., Rhizophagus spp., Septoglomus spp., Funneliformis spp.,* and combinations thereof.; and/or the fungus is selected from: *Trichoderma spp., Trichoderma atroviride, Trichoderma viride, Trichoderma afroharzianum, Paecilomyces spp., Beauveria bassiana., Metarhizium spp., Lecanicillium lecanii, Penicillium spp., Aspergillus spp., Conythyrium minitans, Pythium spp,* and combinations thereof.

As used herein, the nitrogen source is preferably selected from: ammonium phosphates, ammonium nitrate, ammonium sulfate, ammonium thiosulfate, potassium thiosulfate, ammonia, urea, nitric acid, potassium nitrate, magnesium nitrate, calcium nitrate, sodium nitrate, protein hydrolysates of vegetal and animal origin, aminoacids, proteins, yeast lysate, manganese nitrate, zinc nitrate, slow-release urea, preferably urea formaldehyde, similar compounds and combinations thereof.

As used herein, the phosphorus source is preferably selected from: ammonium phosphates, potassium phosphates, phosphoric acid, sodium phosphates, calcium phosphate, magnesium phosphate, rock phosphate preferably hydroxyapatite and fluorapatite, phosphorous acid, sodium phosphite, potassium phosphite, calcium phosphite, magnesium phosphite, organic phosphorus compounds, preferably inositol-phosphate, sodium glycerophosphate, ATP, similar compounds, and combinations thereof.

As used herein, the potassium source is preferably selected from: potassium acetate, potassium citrate, potassium sulfate, potassium thiosulfate potassium phosphate, potassium phosphite, potassium carbonate, potassium chloride, potassium hydroxide, potassium nitrate, mixed salts of magnesium and potassium, potassium sorbate, potassium ascorbate, organic forms of potassium, and combinations thereof.

As used herein, the magnesium and/or calcium source is/are preferably selected from: magnesium nitrate, magnesium sulfate, magnesium chloride, magnesium phosphate, magnesium phosphite, magnesium thiosulfate, magnesium hydroxide, magnesium oxide, mixed salts of potassium and magnesium, mixed salts of magnesium and calcium (dolomite), magnesium acetate, magnesium citrate, magnesium sorbate, and organic forms of magnesium, magnesium carbonate, magnesium formate, magnesium ascorbate, and combinations thereof.

As used herein, the sulfur source is preferably selected from: sulfuric acid, sulfates, thiosulfate, sulfated aminoacids, and combinations thereof.

As used herein, the iron and/or manganese and/or zinc and/or copper source are preferably selected from: iron sulfate, iron oxide, iron hydroxide, iron chloride, iron carbonate, iron phosphate, iron nitrate, chelated iron with EDTA, DTPA, HEDTA, EDDHA, EDDHSA, EDDHCA, EDDHMA, HBED, EDDS; complexed iron with aminoacids, lignosulfonates, humic acid, fulvic acid, gluconic acid, heptagluconic acid, citrate, malate, tartrate, acetate, lactate, ascorbate, organic form of iron, and combinations thereof.

As used herein, the "Plant Growth Regulators (PGRs)" are defined as small, simple chemicals produced naturally by plants to regulate their growth and development. Preferably PGRs are selected from the hormones or phytohormones belonging to the group of auxins, gibberellins (GA), abscisic acid (ABA), cytokinins (CK), salicylic acid (SA), ethylene (ET), jasmonates (JA), brassinosteroids (BR), strigolactones and combination thereof.

In preferred embodiment, the biopesticides (PPP) are selected from: biocontrol products, fungicides, insecticides, herbicides, nucleic acid-based biopesticides, preferably RNA-based or RNA interference (RNAi) based biopesticides as disclosed above.

Preferably said fungicides is selected from: the group of benzimidazoles, preferably selected from: benomyl, thiophanate methyl, thiabendazole and combination thereof; and/or the group of triazoles carboxamide, preferably selected from: ethaboxam, cymoxanil, and combination thereof; and/or the group of triazoles, preferably selected from: cyproconazole, difenoconazole, fenbuconazole, flutriafol, metconazole, ipconazole, myclobutanil, propiconazole, prothioconazole, tebuconazole, tetraconazole, triadimefon, triadimenol, triticonazole, and combination thereof; and/or the group of strobilurins, preferably selected from: azoxystrobin, famoxadone, fenamidone, fluoxastrobin, kresoxim-methyl, mandestrobin, picoxystrobin, pyraclostrobin, trifloxystrobin and combination thereof; and/or the group of ethylenebisdithiocarbamates, preferably selected form: mancozeb, maneb, metiram and combination thereof; and/or the group of EBDC-like, preferably selected from: thiram, ziram, ferbam and combination thereof; and/or the group of aromatic hydrocarbon, preferably selected from dicloran (DCNA), etridizole, pentachloronitrobenzene, RNA-based or RNA interference (RNAi) based fungicides, and combination thereof.

Preferably said insecticide is selected from the group of: Acetylcholinesterase (AChE) inhibitors preferably selected from: Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC, Xylylcarb and combination thereof; and/or organophosphates, preferably selected from: Acephate, Azamethiphos, Azinphosethyl, Azinphos-methyl, Cadusafos, Chlorethoxyfos, Chlorfenvinphos, Chlormephos, Chlorpyrifos, Chlorpyrifosmethyl, Coumaphos, Cyanophos, Demeton-S-methyl, Diazinon, Dichlorvos/ DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothiophosphoryl) salicylate, Isoxathion, Malathion, Mecarbam, Methamidophos, Methidathion, Mevinphos, Monocrotophos, Naled, Omethoate, Oxydemetonmethyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon, Vamidothion; Sodium channel modulators preferably selected from: Acrinathrin, Allethrin, d-cis-trans Allethrin, d-trans Allethrin, Bifenthrin, Bioallethrin, Bioallethrin-S-cyclopentenyl isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-cypermethrin, zeta-Cypermethrin, Cyphenothrin, (1R)- trans- isomers], Deltamethrin, Empenthrin (EZ)- (1R)- isomers],Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-transisomer], Prallethrin, Pyrethrins (pyrethrum), Resmethrin, Silafluofen, Tefluthrin, Tetramethrin, Tetramethrin [(1R)-isomers], Tralomethrin, Transfluthrin, and combination thereof; Nicotinic acetylcholine receptor (nAChR) agonists preferably selected from: Acetamiprid, Clothianidin, Dinotefuran, Imidacloprid, Nitenpyram, Thiacloprid, Thiamethoxam; Inhibitors of chitin biosynthesis preferably selected from: Bistrifluron, Chlorfluazuron, Diflubenzuron, Flucycloxuron, Flufenoxuron, Hexaflumuron, Lufenuron, Novaluron, Noviflumuron, Teflubenzuron, Triflumuron, RNA-based or RNA interference (RNAi) based-insecticides, and combination thereof.

Preferably the herbicide belongs to the class of seedling shoot growth inhibitors, more preferably to the class of the acetamide herbicides or long-chain fatty acid inhibitors, preferably selected from: metolachlor, dimethenamid, napropamide, pronamide and acetanilide herbicides such as acetochlor, alachlor, butachlor, butenachlor, delachlor, diethatyl, dimethachlor, mefenacet, metazochlor, pretilachlor, propachlor, propisochlor, prynachlor, terbuchlor, thenylchlor and xylachlor, mixtures thereof and stereoisomers thereof. Herbicides may be also RNA-based, or RNA interference (RNAi) based.

According to a further preferred embodiment of the invention, the herbicide belongs to: the class of amino acid synthesis inhibitors, preferably to the class of ALS (AcetoLactate Synthase) inhibitors and/or EPSP (5-EnolpPyruvyl-Shikimate 3-Phosphate) synthase inhibitors, preferably glyphosate or derivatives thereof;the class of Lipid Synthesis Inhibition selected from ACCase inhibition Aryloxyphenoxypropionates (FOPs) clodinafop propargyl, diclofop, fenoxaprop, fluazifop-P, Fusilade DX, pinoxaden, quizalofop-P, Cyclohexanediones (DIMs) clethodim, sethoxydim, F tralkoxydim; theclass of growth regulator, Synthetic auxins 1, Phenoxyacetic acids 2,4-D, 2,4-DB, dichlorprop, MCPA, Mecoprop , Benzoic acids, Dicamba, Pyridine carboxylic acids aminopyralid, copyralid, fluroxypyr, F picloram, R triclopyr, Quinoline carboxylic acids quinclorac, Pyrimidine carboxylic acids aminocylopyrachlor.

According to a preferred embodiment, the small RNAs extract from said donor plant is characterized by the presence of one or more of the following miRNAs: miR4995 and/or miR159, wherein miR159 is preferably miR159a-3p and/or miR159e-3p, and/or miR4371, preferably miR4371b, and/or miR482, preferably miR482-5p. For convenience we name said one or more, preferably the plurality of miRNAs, as miRNA profile of the small RNA extract. For the purpose of the present invention miR4995 is the most preferred characterizing miRNA of the extract.

Preferably, miR4995 is the top expressed miRNA of said profile. Preferably, miR4995 and miR159 are the top two expressed miRNAs of said profile.

The following miR159 family members are the preferred: miR159a-3p and/or miR159e-3p and when both are present miR4995, miR159a-3p and miR159e-3p are the top three expressed miRNAs of said profile.

Preferably miR4995, miR159 and miR4371 are the top three expressed miRNAs family of said profile.

miR4371 is preferably miR4371b. Therefore, preferably miR4995, miR159 and miR4371b are the top three expressed miRNAs of said profile.

Preferably miR4995, miR159a-3p and miR159e-3p miR4371 the top four expressed miRNAs of said profile.

According to a preferred embodiment, the small RNAs extract is characterized by an expression level of miR4995 higher than the expression level of miR482, preferably higher than the expression level of miR482-5p. Preferably the ratio between the relative expression level (amount/quantity), or miRNA steady-level of miR4995 and miR482-5p (miR4995/miR482-5p), is greater or equal to 0.5, preferably greater or equal to 1, more preferably greater or equal to 50, still preferably greater or equal to 100.

According to a further preferred embodiment, the expressed miRNAs, preferably top expressed, of the tested extracts of the present invention are preferably the following: miR4995, miR159, miR4371, miR169, miR156, miR167, miR164, miR1511, preferably in the sequence or in any combination thereof. In other words, the small RNAs extract from the donor plant is characterized by a composition or a panel or a signature or a plurality or a profile of miRNAs preferably selected from: miR4995, miR159, miR4371, miR169, miR156, miR167, miR164, miR1511, preferably in the sequence or in any combination thereof wherein, miR159 is preferably miR159a-3p and/or miR159e-3p; miR4371 is preferably miR4371b; miR169 is preferably miR169a, miR169f, miR169g, miR169m, or miR169e; miR156 is preferably miR156k, miR156n, miR156o, miR156a, miR156h, or miR156u; miR167 is preferably miR167g, miR167c, miR167j, miR167a, miR167b, miR167d, or miR167e; miR164 is preferably miR164b, miR164c, miR164d, miR164a, miR164e, miR164f, or miR164g.

The miRNAs profile of the extract has been determined and can be preferably determined by using a genome-wide microRNA (miRNA) expression profiling on GeneChip miRNA 4.0 Arrays and subsequent bioinformatic analysis by aligning the sequences preferably on the *Glycine* max annotated miRNAs in miRBase (as updated at the filing date).

In this context miRNA family is a group of miRNAs that derive from a common ancestor. Generally speaking, members from the same miRNA family have similar physiological functions; however, they are not always conserved in primary sequence or secondary structure. miRNA families are important because they suggest a common sequence or structure configuration in sets of genes that hint to a shared function. miRNA genes in a family can exhibit full conservation of the mature miRNA or partial conservation of only the seed sequences at specific positions of the functional mature miRNA.

Preferably miR4995 has SEQ ID NO: 1 or sequences having 80-99% identity (AGGCAGUGGCUUGGUUAAGGG). Preferably miR159a-3p has SEQ ID NO: 2 or sequences having 80-99% identity (UUUGGAUUGAAGGGAGCUCUA). Preferably miR159e-3p has SEQ ID NO: 3 or sequences having 80-99% identity (UUUGGAUUGAAGGGAGCUCUA). Preferably miR4371b has SEQ ID NO: 4 or sequences having 80-99% identity (AAGUGAUGACGUGGUAGACGGAGU). Preferably miR482-5p has SEQ ID NO: 5 or sequences having 80-99% identity (GGAAUGGGCUGAUUGGGAAGCA).

The sequences of these members can be found in miRBase database which latest version - the release 19 (R19) - contains 21,264 experimentally validated miRNA genes expressing 25,141 mature miRNAs in 193 species.

According to a preferred embodiment, at least one small RNAs extract from at least one donor plant is applied at a concentration lower than or equal to 50 g/ha, preferably lower than or equal to 5 g/ha, more preferably lower than or equal to 0.5 g/ha, still more preferably lower than or equal to 50 mg/ha, wherein this concentration is referred to the small RNAs concentration per hectare.

Therefore, for example if the small RNAs enriched extract from a donor plant is 5% titration, that is 5% enriched/concentrated compared to the total extract, this means that we have to use 100 g of the extract 5% enriched of small RNAs per hectare to apply 5g/ha of small RNAs, or that we have to use 10 grams of the extract 5% enriched of small RNAs per hectare to apply 0.5 g/ha of small RNAs, or that we have to use 1 g of the extract 5% enriched of small RNAs per hectare to apply 50 mg/ha of small RNAs. If the titration of the extract is 10% of small RNAs, the quantity to be used is half.

According to a preferred embodiment the application of the extract may be done according to the conventional agricultural ways, preferably by spraying, soil drench, root drench, in furrow, or via seed treatment, seed soaking, post-harvest treatment and combination thereof.

According to a preferred embodiment of the invention, the application of the extract may be done according to the receiving plants, that is the crop. Preferably, for row crops it is advisable to perform one application at the dosage disclosed above, while for vegetable and/or orchards it is preferable to have 1-3 applications at the dosage disclosed above. The application may be done in the vegetative (V) as well as in the reproductive (R) phase of the crop as needed or as requested based on the crop, preferably the application is 1-2 for the row crops, preferably 3-4 applications for vegetable and orchards. Moreover, the application depends upon the agronomic target of interest, for example for improving yield and/or for improving nutrient use efficiency in corn it is preferable to perform the application at V3-V5 and/or V5-V6 and/or R1 wherein these phases are well known to the expert in this field.

The spray application is preferably done in field directly on the plants/crops to be treated. In the preferred embodiment, the extract is provided alone as such preferably as concentrate extract which is diluted with water upon filling a spray tank. Preferably the concentrated extract is lyophilized or in dry form.

Alternatively, the extract is formulated as disclosed above with adjuvants and it can be a single formulation, meaning that the extract of small RNAs and the adjuvants are mixed in one single formulation; alternatively, the extract of small RNAs and the adjuvants can be mixed immediately prior to application to the receiving plants in the field.

### EXAMPLES

The following examples relate to the results obtained by testing several extracts enriched of small RNAs, wherein the miRNAs have a length that is less than 200nt, in particular ranging between 19-25 nucleotide (nt) (from now on these extracts are simply named "the extracts" or "the extracts of the invention").

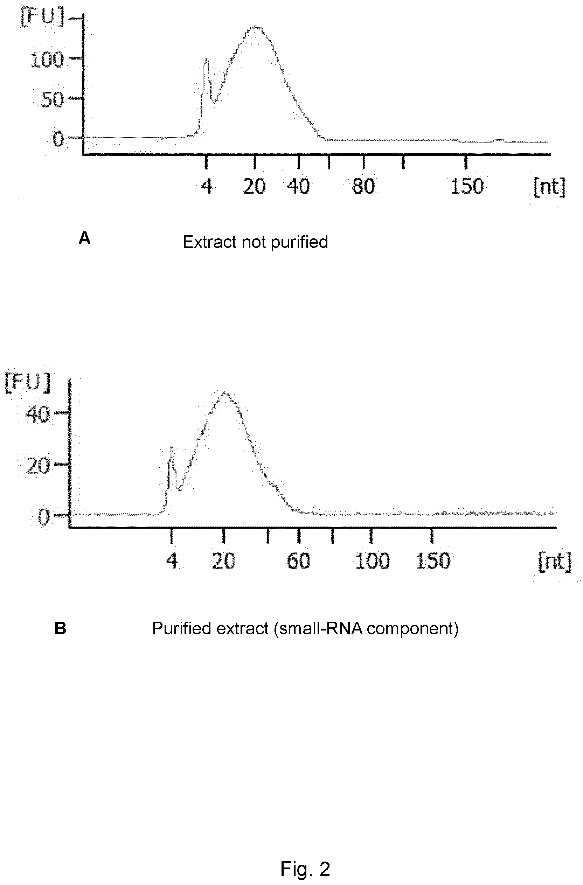

Extracts were prepared starting from different matrices/plant materials, such as several Leguminosae such as peas, soybean, beans, alfa alfa, and others. The extracts were then tested for several agronomic biological activities and effects. The typical electropherogram at the Bioanalyzer of the extracts prepared and tested are showed in Fig.2.

The extracts were prepared by using several protocols known for this purpose. In particular, in these examples the extracts tested for potential agronomic effects have been obtained from a process that involves treating the plant or part thereof (the starting matrix/material from which smallRNAs/miRNAs have to be extracted) for 1-12 hours with bicarbonate solution at a temperature of 50-100 °C and then precipitating the small RNAs with ethanol.

Below some of the results of the tests performed are reported; these tests have been done by using some of the extracts from different matrices, named as Extract A, Extract B etc.

Several doses (dosages) or application rates of the extracts were tested on different crops of interest. The dose of application or application rate is mentioned for in-field trials and is expressed as dose of active ingredient (Al) per hectare (Al Dose/ha) wherein in this case the Al is meant for the small RNAs content (not the active extract which is the extract comprising the small RNAs). In this context, it should be considered that the volumes applied per hectare may vary based on several parameters e.g., the geography, crop, phenological stage of the crop etc.

Besides small RNAs title quantification, the tested extracts have been profiled for miRNAs by genome-wide microRNA (miRNA) expression profiling on GeneChip miRNA 4.0 Arrays and subsequent bioinformatic analysis by aligning the sequences on the Glycine Max annotated miRNAs in miRBase (as updated at the filing date).

The top common miRNAs expressed in the tested extracts are the following: miR4995, miR159, miR4371, miR169, miR156, miR167, miR164, miR1511.

The extracts have been also characterized chemically and overall, they share the following composition: small RNAs + carbohydrates + inorganic matter + small molecules and/or natural metabolites and/or relative by-products.

The small RNAs, which is for us the Active Ingredient (Al) as shown herein, are characterized by a length <200 ribonucleotides (nt), with the highest proportion having a size that ranges between 21 and 24 nt, and in a titration up to 20%, and as average a titration ranging between 5 to 10%; the carbohydrate component is present as average 1-15%; the inorganic matter is present as average 10-30%; the small molecules and/or natural metabolites and/or relative by-products is present as average 25-40%. The most homogeneous class of molecules of the extracts are the small RNAs, while the remaining classes of molecules are heterogeneous and belong to the following families: peptides, free amino acids, oligosaccharides, natural chelating agents, cofactors, nucleobases, saponins and phenols.

### SMALL RNA TEST - VERTICAL PLATE

The extracts were tested through the *in vitro* Vertical Plate bioassay which in this context has been used for a rapid evaluation of a phenotypically evident biological effect/potential/induction. For this purpose, *A.thaliana* plants (wild type, ecotype Columbia-0) were grown on vertical plates agar media. Sterilized seeds (10/plate) were sown on 0,7% (w/v) agar half-strength Murashige and Skoog medium and 0.5 % of sucrose, supplemented with the extracts to be tested and stored in the dark for 48h at 4°C before being transferred at 23°C with a 12-h light photoperiod (100 mE m2 s2 intensity). The primary root length of the plants has been measured after 7-10 days from the seed's germination, while the fresh and dry plant biomass have been evaluated after 15 days from the seed's germination (roots and shoots).

The extracts have been tested in a broad range of concentrations ranging from 0.01mg/L to 100mg/L.

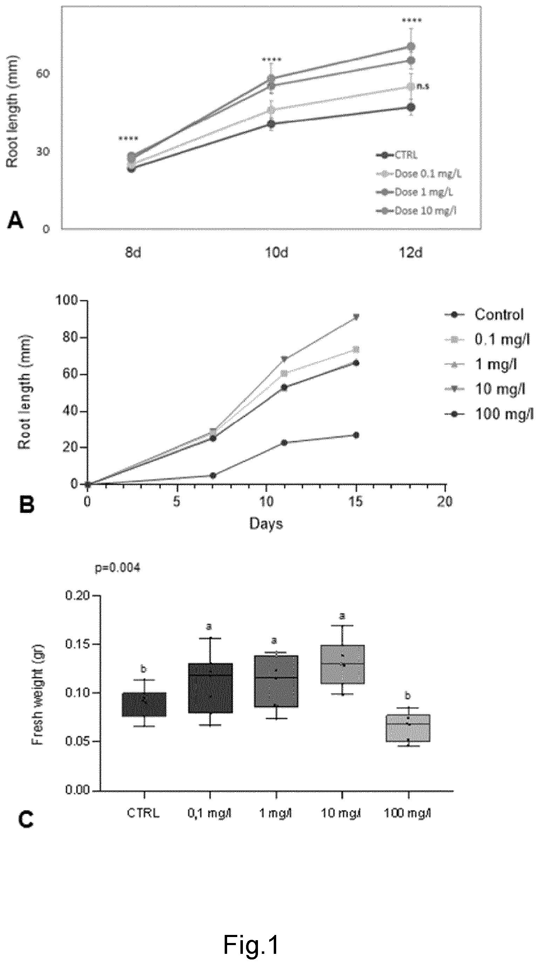

Fig. 1 shows the effect of Extract B at 0.1, 1, and 10 mg/L of small RNAs on the plant root growth. Results show that the length of the root as well as its biomass (Fig.1C) increase with the concentration of the applied small RNAs. Similar results were obtained also with further extracts (data not shown) and have been repeated several times.

We have tested the small RNA-enriched extracts also at higher concentrations and have observed a drop in the positive biological effect observed on plant root growth at 100mg/L. In other words, there is a modification of the positive correlation between the application of small RNAs containing extract starting from 100mg/L of small RNAs applied as extract (Fig. 1-B, C).

We performed tests with the extracts after RNase treatment and purification of the small RNAs fraction by using kits known for the purpose. To this end, *in vitro* vertical plates bioassays were performed according to the protocol reported above. The treatments were the following:
(i) the extract containing the small RNAs as prepared above (the positive control);
(ii) the small RNAs portion of the extract purified by using a small RNAs extraction kit according to the manufacture instruction (Qiagen RNeasy Plant Mini Kit);
(iii) the small RNAs enriched extract treated with RNAse A to degrade the small RNAs component.

The purified small RNAs were quantified through a Qubit fluorometer (2.5-5 and 10 mg/L).

Extracts (i) (ii) (iii) above were used to supplement the vertical plates with different concentrations of Al. The plates were stored in the dark for 48h at 4°C before being transferred at 23°C with a 12-h light photoperiod (200 mE m⁻²s⁻² intensity).

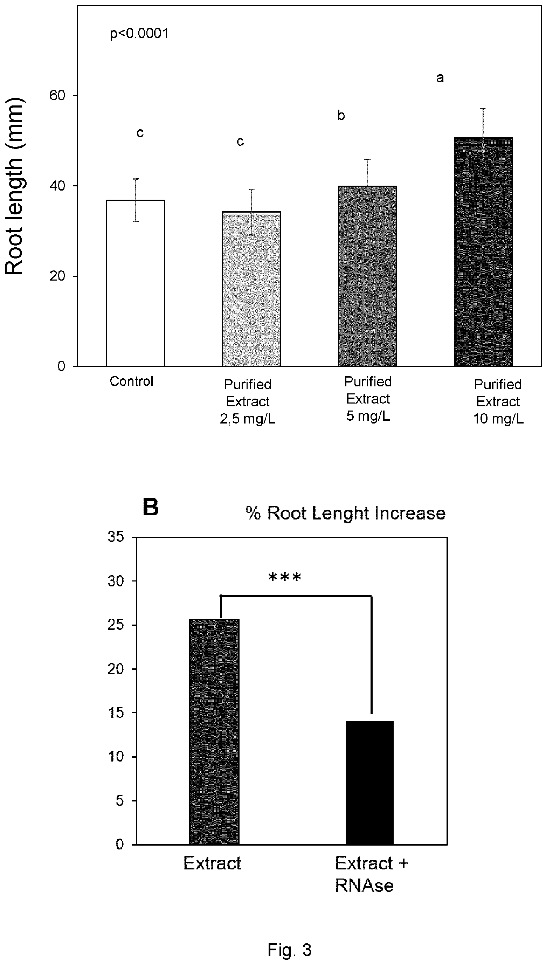

The results for vertical plates are shown in Fig.3 and demonstrate that all tested concentrations resulted in the increase of the plant root length, and the dose 10 mg/L induced the most remarkable effect (Fig.3A).

When the plant growth media was supplemented with the small RNAs extract, previously treated with RNAse A, the positive effect on the plant root growth is impaired, thereby demonstrating that the small RNAs are, directly or indirectly, involved in the modulation of root growth (Fig.3B).

### DATA ON CORN

### Corn seed germination assay

In view of the vertical plate outcomes on plant root growth and biomass, a potential positive effect on plants following the extract's application (the small RNAs component being the Al) in particular on the nutrient uptake and on crop yield and growth was supposed.

Considering the economic relevance, as well as the impact on human nutrition of row crops, we moved from a bioassay using *A.thaliana* (vertical plate) to an *in vivo* bioassay closer to the crops of major commercial interest. As an example, we report herein the results of the "Seed Germination Pouch" (SGP) assays performed on corn as plant model. SGP is a space-saving system in which root growth can be observed visually; seeds of corn plants (*Zea mays* L., var. *Corniola*) were sowed in a medium pouch (PHYTO AB, CA, USA) inside the through formed by the paper wick. The SGP was then placed in an upright position using a stand, and 10 ml of sterilized distilled water was added. Plants were grown inside a growth cabinet (PERCIVAL) set with the following parameters: 16h day, 24 °C, light intensity 300 µmol m⁻²s⁻¹. Throughout the experiment, water was controlled and added, as needed. After seven days from sowing, 24 pouches containing uniform plants were selected. The experimental setup was composed of 8 replicates (pouches) for each experimental condition (treatments) using a completely randomized design.

Different concentrations of extract were tested and results demonstrate a positive effect both in terms of main plant biological parameters such as root length, weight, structure as consequence of the application of the extracts at all tested concentrations.

As for the vertical plate bioassay, to prove that the small RNAs component of the extracts are the Al, the small RNAs component was purified with the specific kit according to the manufacture instruction (Qiagen RNeasy Plant Mini Kit). The small RNAs purified were then analyzed by the Bioanalyzer to confirm the expected electropherogram peak (Fig 2A, B). The small RNA containing extract and the purified small RNA were tested on SGP as explained above by foliar application at 1 mg/L rate using a 50ml-spray bottle. The untreated control plants were sprayed with water.

Four days after treatment, data on Shoot Length (SL), Shoot Weight (SW), and Root Weight (RW) were collected. The statistical analysis and significant differences among means were evaluated by ANOVA (p < 0.05) using "Statistica 12" software and means compared by standard Newman-Keuls test.

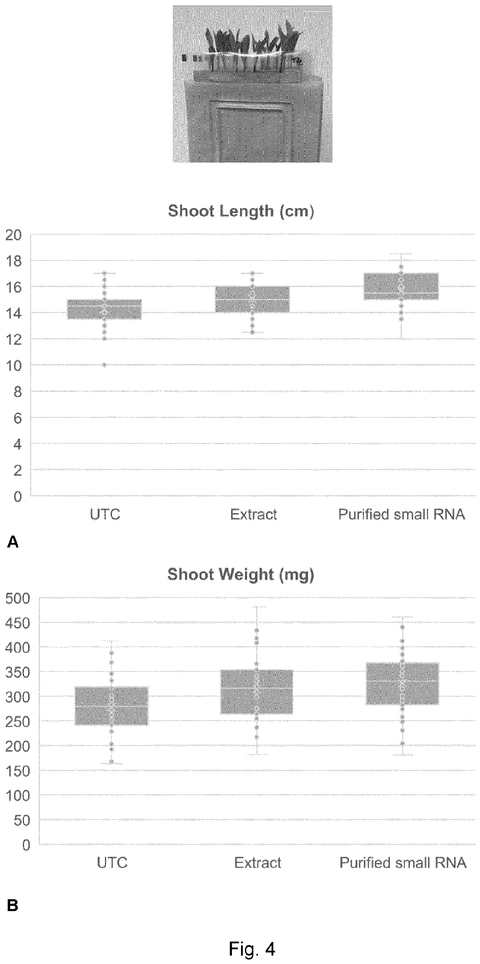

The results are reported below in Table E and in Fig.4A and B, and confirmed the specific, positive, and significant effects induced by the purified small RNA component of the extract on the shoot weight and length, which indeed were significantly increased, compared to the control (p<0.05) and, more importantly, the increase is comparable to the effect induced by the not purified extract.

**Table E**

| **TREATMENT** | **SL (cm)** | **SNK (p<0,05)** | **SW (mg)** | **SNK (p<0,05)** | **RW (mg)** | **SNK (p<0,05)** |
|---|---|---|---|---|---|---|
| UTC | 14.3 | b | 277.5 | b | 732.9 | b |
| Extract (1 mg/L) | 15.1 | a | 314.4 | a | 824.4 | a |
| Purified (1 mg/L) | 15.7 | a | 326.6 | a | 791.8 | ab |

Moreover, we used SGP to test the total smallRNAs extract at the following concentrations: 1mg/L, 10mg/L, 100mg/L and - as for the vertical plate results - we observed a decrease of the biological activity measured in term of shoot weight, shoot length, root weight and root area at 100 mg/L (data not shown).

### Phenomic test - Plant Material and Growing Conditions

The small RNAs extracts were tested through a phenomics approach based on multi-spectrum image analysis to detect morpho-physiological parameters following extracts application.

Corn seeds were pre-germinated in Petri dishes covered with filter paper and humidified with deionized water. After 72 hours, uniformly germinated seeds were selected and transferred into plastic pots containing 1.5 kg of a substrate consisting of a 50:50 mixture of peat and river sand.

Plants were grown in a greenhouse under natural light conditions at the Plant Phenomics Platform, ALSIA-Metapontum Agrobios Research Center, Italy (N 40°23' E 16°47'). During the entire experiment, all plants were fertilized every twenty days by using an NPK fertilizer ("Slowenne", Valagro SpA) for a total of 15 grams per pot. Starting from the seedling phase, pots were irrigated daily, keeping the soil moisture at field capacity.

On day 0 (the beginning of the experiment), corn plants were at the V4-V5 growth stage, and 72 plants were grouped to define 9 treatments. The experimental setup was composed of 8 biological replicates (plants) for each experimental condition (treatments) using a completely randomized experimental design.

The extract was foliarly applied, using a portable atomizer sprayer, at the following rates of the small RNAs component: 0.1 mg/L, 1 mg/L, and 10 mg/L. The commercial product YieldOn^{®} Valagro was applied at a rate of 5mL/L as a positive control of the experiment (corresponding to 2 L/ha). The untreated control plants were sprayed contextually with water. For all conditions, a final spray solution volume of 500 L/ha was considered.

Four treatments out of nine were sprayed 3 times every 7 days during the experiment (starting from day 0). The remaining treatments were sprayed only once, at the beginning of the experiment (day 0).

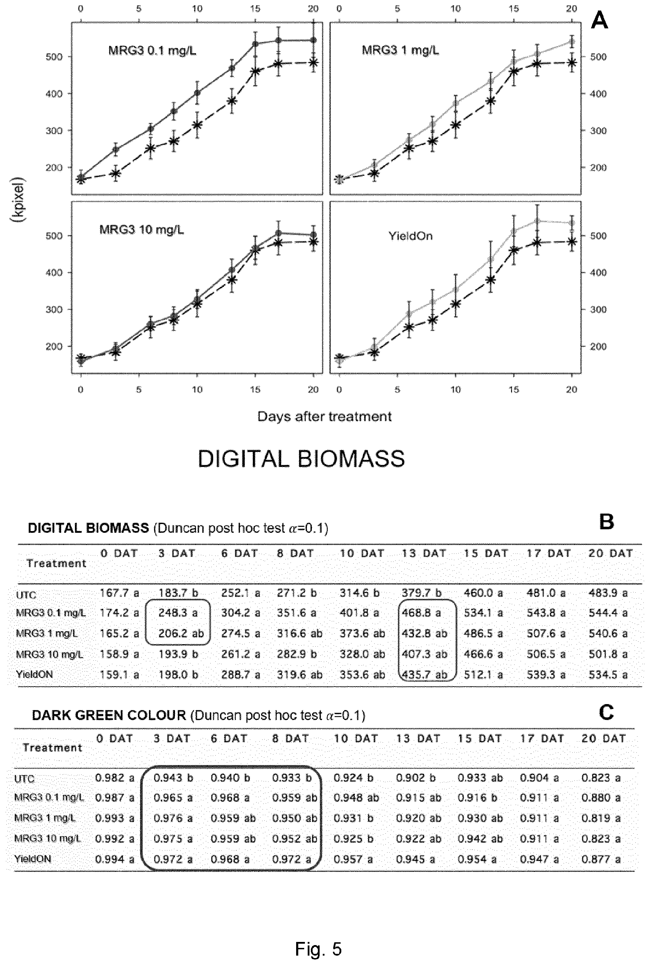

For convenience here, the results on Digital Biomass (DB - Fig. 5A, B), Fresh Weight (FW), Plant Height (PH), and Dark Green color (DG- Fig. 5C) are discussed in more detail. The most relevant results were observed at 0.1 and 1 mg/L, either with single or multiple spray applications for all parameters. Indeed, corn plants treated with a single application of the extract at 0.1 and 1 mg/L of small RNAs showed, starting from the 3^{rd} Day After Treatment (DAT) and 10^{th} DAT, respectively, a statistically significant increase in DB compared to the Untreated Control (UTC) plants. Interestingly, the plants treated with the extract at 0.1 mg/L of small RNAs maintained greater DB (average of values +21.7%), compared to the UTC ones, throughout the experiment. Plants treated with the extract at 1 mg/L showed, at the end of the experiment (20 DAT), a DB increased by +11.8% compared to the UTC plants (Fig.5B).

FW data were collected fifty days after the end of the imaging acquisition period, and the results are consistent with that of DB (statistically significant increase of FW +14.4%, +32.5%, +44.8% *vs.* UTC). Even PH was positively influenced by the application of the extract (e.g. +17% *vs*. UTC starting from 6^{th} DAT to 17^{th} DAT). DG values decrease over time and lead to senescence and yellowing. Interestingly, corn plants treated with the extract showed stable DG values (average of +2.6% increment from 3 DAT to 10 DAT compared to the UTC - single as well multiple applications, Fig.5C).

### Greenhouse trial: Plant Material and Growing Conditions

We also performed Greenhouse (GH) tests on corn plants (*Zea mays* L., var. Corniola). To guarantee the uniformity of plants, three corn seeds were sowed directly into the pots (17 cm diameter, 20 cm height) and humidified by using a drip irrigation system (fertigation plant). After 10 days, one plant for each pot was selected, containing 1.5 kg of a substrate consisting of a 50:50 mixture of peat and river sand.

The experiments were conducted in a glasshouse under natural light conditions at Valagro HQ, Italy (N 42°13' E 14°43'). During the entire investigation, all plants were fertilized every two days using an NPK water-soluble fertilizer ("Master", Valagro SpA) and two ammonium nitrate applications, for a total of 3 grams of fertilizer per pot. From the seedling phase until the end of the experiment, all pots were irrigated daily, keeping the soil moisture at field capacity, and no pest control was applied. The experimental conditions (11 in total) were arranged in a completely randomized block design, made of ten blocks, each one consisting of six plants.

For all treatments, a single application was performed at vegetative phase V3-V4 (BBCH index: 13-14), considering a final solution of 300 L/ha. The extract was applied foliarly by using a portable atomizer sprayer. The commercial product YieldOn^{®} Valagro was applied as a positive control of the experiment, used at a rate of 6 mL/L (corresponding to 2 L/ha). The UTC plants were sprayed contextually with water.

Ten days after the treatment, the following parameters were measured: Plant Height (PH), Plant Biomass (PB), and Green Index (SPAD value). The data collected were subjected to analysis of variance (ANOVA) using "Statistica Software" and statistical analysis, using "Duncan's" tests at different p-value (p<0.05 and p<0.10). Below in Table F the results with Duncan's test at p<0.01 are summarized.

**Table F**

| **Treatment** | **Dosage** | **Fresh weight (g)** | **P<0.01** | **Height (cm)** | **P<0.01** | **SPAD value** | **P<0. 01** |
|---|---|---|---|---|---|---|---|
| Untreated Control | | 9.8 | bc | 34.2 | c | 35.0 | c |
| Extract | 1.67 mg/L | 10.6 | abc | 36.0 | abc | 37.2 | ab |
| Extract | 16.67 mg/L | 11.2 | ab | 37.0 | abc | 36.1 | abc |
| Extract | 166.67 mg/L | 10.7 | abc | 35.7 | bc | 35.9 | abc |
| Extract + coformulants | 1.67 mg/L + co-formulant | 10.9 | ab | 35.9 | abc | 37.2 | ab |
| Extract + coformulants | 16.67 mg/L + co-formulant | 11.4 | a | 36.9 | abc | 36.0 | abc |
| Extract + coformulants | 166.67 mg/L + co-formulant | 10.4 | c | 37.8 | abc | 36.8 | abc |
| 3-2577 (YieldOn^{®}) | 2 L/ha | 11.8 | a | 40.1 | a | 36.4 | abc |

In general, a statistically significant positive effect of the extract on all parameters collected was observed. Corn plants treated with the extract showed a statistically significant increase in PB and PH compared to the Untreated Control (UTC) plants. In particular, the rate 16.67 mg/L was effective (+16.3% *vs.* UTC) on PB when combined with the co-formulants, while the application at the rate of 1.67 mg/L exerted a positive effect on DB (+16.7% *vs.* UTC).

Moreover, the plants treated with the extract at 1.67 mg/L alone or with co-formulants show a statistically significant increase in the Green Index (SPAD value).

### Open field trials on corn

After GH trials, tests of the extracts moved in field to confirm that the agronomic performance demonstrated in laboratory and semi-field was confirmed in these conditions. Plants (corn hybrid P2088) of 130 days were treated with the following dosages (application rate) of small RNAs component of the extract: 0.1 mg/L (D1), 1 mg/L (D2) and 10 mg/L (D3). We tested several extracts, and here we report as examples the results we obtained with Extract A and B.

The study design was a randomized complete block with the following features: No. of entries = 6; No. of Replicates = 6; No. of plots = 6 x 6 = 36. Each plot was 15 sqm [3m (4 rows) x 5m length. The nutrition plan adopted was 100% nutrition. Products were applied at foliar level 3 times at 500 L/ha water volume, with a 10-12-day interval, starting around the flowering stage (R1). Two irrigation events were performed. Duration of the trials 6 months. The corn yield was calculated considering the weight of 10 cobs per row, for a total of 20 cobs for each plot. Moreover, the standard moisture of 14% was considered to obtain the final values. The statistical analysis was carried out considering one value for each plot. This value resulted from the average of the two central rows of every plot.

The results are summarized on Table G and show a very surprising and great increase (overall 10%) in yield (statistically significant p<0.05). Same good results are observed for many key agronomic parameters for this crop, such as fresh biomass and total plant biomass, number of seeds per cob. Interestingly, even in open field a dose response effect was observed wherein the lowest concentration (D1) is the most performant.

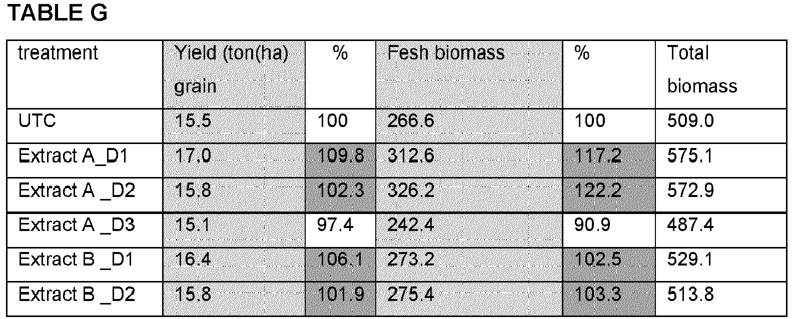

Micronutrients and macronutrients analysis were performed on leaves of corn to verify any impact of the small RNA component application on nutrients uptake from plants.

Leaf samples were collected from every plot at the second and third day of treatment. Elemental analyses were carried out on the material to quantify total Nitrogen, Boron, Calcium, Iron, Magnesium, Manganese, Potassium, Copper, Zinc, Sulphur and Phosphorus. The statistical analysis was performed by means of ONE-WAY of variance (ONE-WAY ANOVA), using the Statistica software by StatSoft, with Duncan test (α)= 0.05.

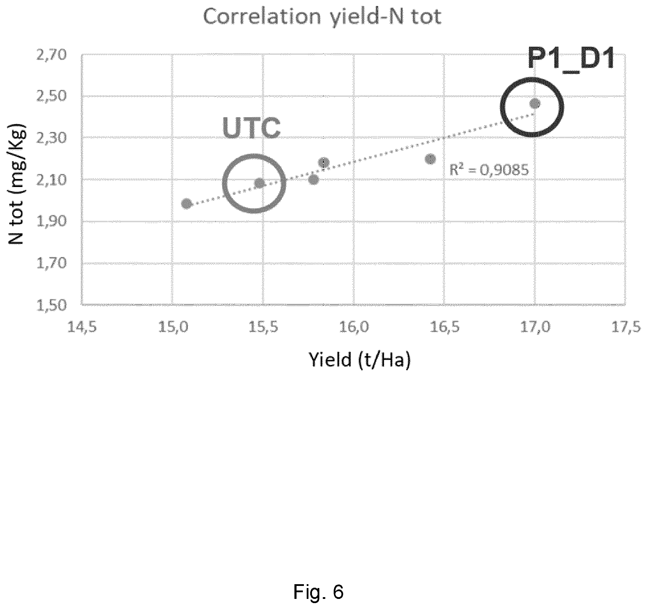

The results show an unexpected increase of the Nitrogen uptake with an increase up to 18,4% compared to untreated plants. The increase was more evident at a lower application rate and total N measured after treatment showed a significant and good correlation with final yield which is the major parameter of interest for growers. Table H and Fig. 6.

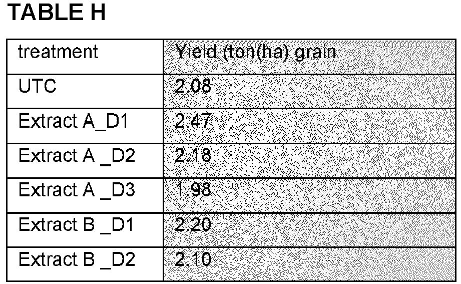

Besides Nitrogen uptake improvement our macro-micronutrients analysis also showed a significant increase of the following elements: Boron, Calcium, Manganese, Iron, Copper and Potassium (data not shown).

### Open field trials on soybean

Plants (var. P21T45 Pioneer) were treated with the following dosages (application rate) of small RNAs: 0.01 mg/L (D1), 0.1 mg/L (D2), 0.3 mg/L (D3), 1 mg/L (D4) and 10 mg/L (D5).

We tested several extracts and here as examples we report the results we obtained with Extract A and B.

The *study design* was a randomized complete block with the following features: *No. of entries* = 40; *No. of Replicates* = 10; *No. of plots* = 40 x 10 = 400. Each plot is 15 sqm [2m (4 rows) x 7.5m length]. Products were applied at foliar level 1 time at 500 L/ha water volume, at three different plant stage: V3, V5 and R1. The same trial was performed in two different sites: SITE 1 and SITE 2. The air temperature and rain events were recorded by means of a weather station. Biological pest control was applied to the crop when necessary. All cultivation measures were performed in accordance with the conventional recommendations for the cultivar growing. Duration of the trials 5 months.

Several assessments were done, and results were very interesting for several parameters (data not shown). Below Tables I and L summarize the most important results in terms of final yield.

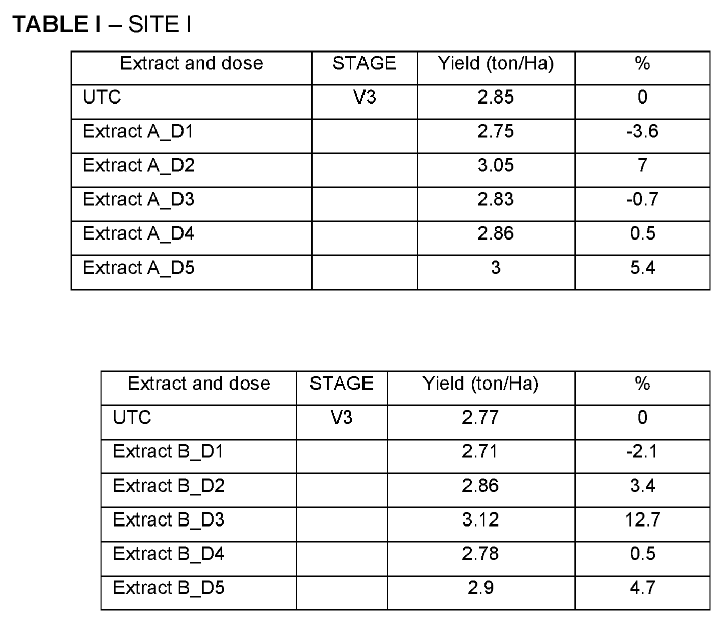

V3 treatment was the most effective; indeed, it determined +7% yield increase with Extract A (0.1 mg/L), and +12.7% a with Extract B (0.3 mg/L).

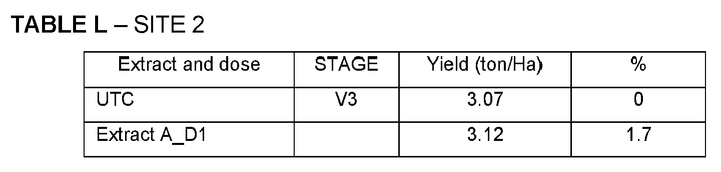

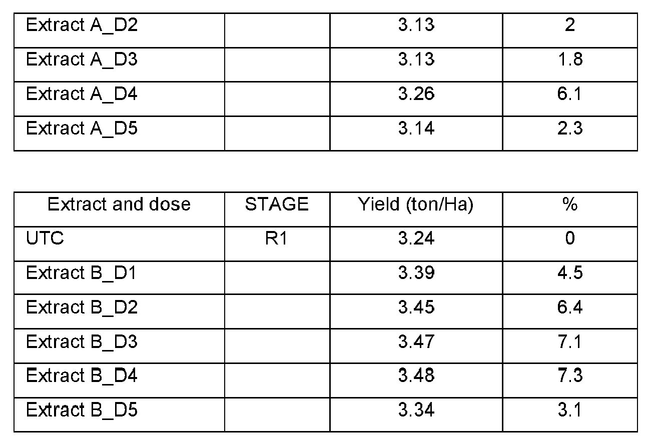

Overall, the best performance was showed by Extract A at V3 and by Extract B at R1 with positive effects at all tested application rates of the extracts.

Micronutrients and macronutrients analysis were performed on leaves and seeds of soybean grown in pots to verify any impact of the small RNA component application on nutrients uptake from plants. Plants were cultivated under full NPK (100%) and with 50% reduction of P and K. The study design was a randomized complete block with the following features: No. of entries = 13; No. of Replicates = 9; No. of pot per replication= 5; No of plots=13x5x9 = 585. Each pot (35 Liter) can contain one row of soybean, 9 seeds. The substrate used was 70% sand and 30% soil. The application was made at foliar level with 500L/ha water volume. At the start of the trial, five soil samples were taken to perform analysis of elements. The air temperature and rain events were collected by using a weather station.

Phosphorus, Potassium Sulfur, Boron, Calcium, Magnesium, and Manganese increased under reduced (50%) nutritional regime both in the plants as well as in the seeds with yield and roots biomass increase correlation. The best performance was measured in seeds.

### Open field trials vegetables (cash crops): Strawberry

Plants (Irma variety) were treated with the following dosages (application rate) of small RNAs: 0.1 mg/L (D1), 1 mg/L (D2) and 10 mg/L (D3). The study design was a randomized complete block with the following features: No. of entries = 6; No. of Replicates = 5; No. of plots = 6 x 5 x 6 = 180.

The trial was in a rain-out tunnel, on specific supports for strawberries, onto 3 L pots filled with peat moss. The setup of the trial included: Pot preparation, placing of the pots on the proper strawberry-supports and transplant of young plants, Biological pest control. Nutrients were applied pot by pot via fertigation with a total of 0.5 g/pot N-P-K. Extracts were applied as foliar treatment three times at 500 L/ha water volume, with a 10-12-day interval, starting from the beginning of the flowering stage. Biological pest control was applied to the crop when necessary. All cultivation measures were performed in accordance with the conventional recommendations for the cultivar growing. Air temperatures were collected and monitored by means of a weather station. Fruits were harvested 5 times during the trial, and the last harvest included green fruits. The color of strawberries was assessed for every harvest. A range from 1 to 5, where 1=pale red and 5=dark red was used to determine the redness of fruits.

Results are summarized in the Table M below and show a good trend in terms of fruit numbers and color.

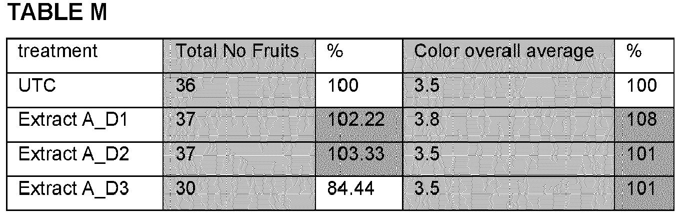

### Open field trials vegetables (cash crops): melon

Plants (Momo variety) were treated with the following dosages (application rate) of small RNAs: 0.1 mg/L (D1), 1 mg/L (D2).

The study design was a randomized complete block with the following features: No. of entries = 7, No. of Replicates = 5 (4 plants each), No. of plants = 7x5=35 plants. The treatments were planned in order to spray the products on the flowering plants and during the fruit setting time. The trial was carried out in large plots, in a private field, where all conventional practices were performed by the farmer. The area was limited, considering the most homogeneous plants. All the selected site was divided into several plots, according to the experimental design. The total number of replicates was reduced by problems that occurred to some plants not properly grown. The products were applied three times by manual spray, with a 10-12-day interval, starting from the flowering time, using a water volume of 500L/Ha. To study the effects induced by the treatment with the extracts on the melon plants' yield, the fruits of treated and untreated plants were harvested, counted and the total weight per plant measured. The results are summarized in Table N below showing overall positive response on key parameters (here reported for convenience fruit number and weight) after extract application , the best results being obtained at a lower application rate (e.g up to +52% increase of fruits No respect to the UTC).

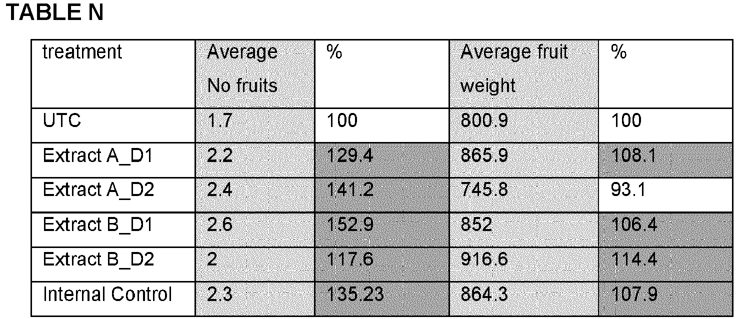

### Open field trials vegetables (cash crops): tomato

Tomato plants were grown on multiwell trays with standard soil as substrate under controlled conditions (T=23/18°C, 12h light/12h dark). Four weeks old plants were sprayed with 10 mg/L and 50 mg/L of extract (the concentration refers to the small RNAs). Two treatments (7 days distant) were performed on many plants (60 plants for each concentration). With the last treatment plants were split in two trays: one regularly watered, and the other one under water stress induced by lack of water supply. The effect on flowering time was monitored by counting the number of flowers per plant starting from the tenth day after the first treatment and for the next eight days.

Results demonstrate a positive effect with increased number of flowers at the tested concentrations compared to the control. The lowest dosage (10 mg/L) shows the best performance since the start of the monitoring in terms of both flowering time and number of flowers per plant. Lowest dosage (10 mg/L) shows analogous results under drought stress condition; however, under this stress higher dosage (50 mg/L) did not result in any significant increasing effects on flowering time and flowers produced per plant.

### Production on Tomato

The products were applied at foliar level 3 times at 500 L/ha water volume, with a 10-12-day interval, starting around the flowering stage. Plants were treated with the following final dosages of extract of small RNAs: 0.1 mg/L (D1), 1 mg/L (D2). The trial was carried out in large plots, in a private field, where all conventional practices were performed by the farmer. The area was selected considering the most homogeneous plants.

The study design was a randomized complete block with the following features: No. of entries = 7, No. of Replicates = 5 (4 plants each), No. of plants = 7x5=35 plants. The trial was setup to investigate the effects of the extracts on tomato processing final yield. After one month from the last treatment, the fruits were harvested, counted and weighted. Results are summarized in Table O below and show overall interesting results in terms of several key parameters for this crop. In particular, the total fruit number/plant is positively influenced by the extract (up to +23,4% increase compared to the untreated control) and even final yield with an up to +28% increase compared to control.

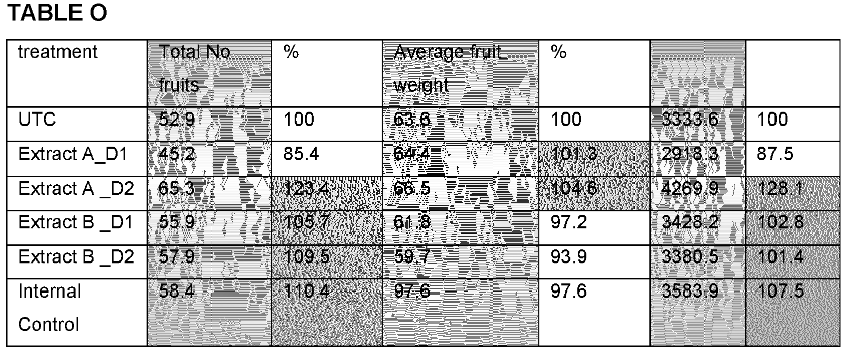

## Claims

1. A plant extract comprising:
(a) up to 21% of small RNAs;
(b) up to 16% of carbohydrates;
(c) up to 36% of inorganic matter; and
(d) the remainder, up to 76%, of small molecules and/or natural metabolites and/or relative by products.

2. The plant extract according to claim 1, wherein the extract comprises:
(a) from about 5 to about 20% of small RNAs;
(b) from about 1 to about 15% of carbohydrates;
(c) from about 5 to about 35% of inorganic matter; and
(d) from about 45 to about 75% of small molecules and/or natural metabolites and/or relative by products.

3. The plant extract according to claim 1, wherein the extract comprises:
(a) from about 5 to about 10% of the small RNAs;
(b) from about 1 to about 10% of the carbohydrate;
(c) from about 10 to about 30% of inorganic matter; and
(d) from about 50 to about 70% of small molecules and/or natural metabolites and/or relative by products.

4. The extract according to any one of claims 1-3, wherein the small RNAs are less than 200 nucleotides in length, preferably 20-25 nucleotides, more preferably 21-24 not or 18-24 nt or 19-22 nt or 21-22 nt.

5. The extract according to any one of claims 1-4, wherein said one or more small RNAsis selected from: miR4995, miR159, preferably miR159a-3p and/or miR159e-3p, miR4371, preferably miR4371b, and combinations thereof.

6. The extract according to any one of claims 1-5, wherein said one or more small RNAs define a profile of molecules selected from: miR4995 as the top expressed miRNA; and/or miR4995 and miR159 as the top two expressed miRNAs; and/or miR4995, miR159 and miR4371 or miR4995, miR159 and miR4371b or miR4995, miR159a-3p and miR159e-3p as the top three expressed miRNAs; and/or miR4995, miR159a-3p, miR159e-3p and miR4371b as the top four expressed miRNAs.

7. The method according to claim 5, wherein the profile further comprise miR482, preferably miR482-5p and wherein the ratio between the relative expression level of miR4995 and miR482-5p (miR4995/miR482-5p) is greater or equal to 0.5, preferably greater or equal to 1, more preferably greater or equal to 50, still preferably greater or equal to 100.

8. The extract according to any one of claims 1-7, wherein the extract comprises one or more of or each of: peptides, proteins, free amino acids, oligosaccharides, natural chelating agents, cofactors, non-RNA nucleobase, saponins, phenols, and combinations thereof.

9. A formulation comprising:
(a) an extract according to any one of claims 1-8; and
(b) an agricultural co-formulant and/or adjuvant.

10. The formulation according to claim 9, wherein the agricultural formulant and/or adjuvant comprises stickers, fillers, binders, surfactants, dispersant, polymers, carriers, pH regulators, UV-protectants, and combinations thereof.

11. The formulation of claim 9, wherein the adjuvant reduces surface tension and/or improves solubilization, spreading, retention, and penetration of small RNAs.

12. The extract according to any one of claims 1-8 or the formulation according to any one of claims 9-11 for use in agriculture, preferably for improving the agronomic performance or trait of a target crop plant.

13. The extract or the formulation for use according to claim 12 applied to the receiving crop plant at a concentration lower than or equal to 50 g/ha, preferably lower than or equal to 5g/ha, more preferably lower than or equal to 0.5 g/ha, still more preferably lower than or equal to 0.05 g/h wherein the concentration refers to the amount of small RNAs in grams per hectare, or applied to the receiving crop plant of step (i) at a concentration lower than or equal to 100 mg/L, preferably lower than or equal to 10 mg/L, more preferably lower than or equal to 1 mg/ha, still more preferably lower than or equal to 0.1 mg/ha wherein the concentration refers to the amount of small RNAs in milligrams per Liter.

14. The extract or the formulation for use according to claim 12 or 13 wherein the receiving crop plant is a monocotyledonous and/or dicotyledonous, preferably selected from:
• Grain crops, preferably cereals or pseudo cereals and legumes or pulses; the cereals are preferably selected from: maize (corn), millet, pearl or proso millet, sorghum, spring wheat or cool-season cereals, preferably selected from: barley, rye, rice, oats, spelt, teff, triticale, wheat and combinations thereof;
• the pseudo-cereals are preferably selected from: buckwheat, starchy grains from broadleaf, amaranth, buckwheat, chia, quinoa, and combinations thereof;
• other row crops, preferably selected from: cotton, sunflower, tobacco, canola, sugar cane, hop, peanut and combinations thereof;
• fruit and vegetable crops, preferably sugar beets, potatoes cabbages, cauliflower, broccoli, radish tomatoes, chicory, processing tomatoes, fresh tomatoes, eggplant, pepper, pumpkin, bell pepper, cucumber, zucchini, onion, garlic, lettuce, leafy vegetable, ginger, alkekengi, tea plant, celery, spinach, asparagus, fennel, berries in general and preferably strawberry, black berries, blue berries, peach, nectarines, apricot, cherry, plum, cedar, alchechengi, apple, pear, citrus, orange, tangerine, mandarin, clementin, satsuma, lemon, lime, pear, melon, watermelon, cucumbers, onion, shallot, artichoke, lettuce, spinach, beets, carrots, table grape, wine grape, olive, kiwifruit, almond, walnut, hazelnut, pomegranate, and a combinations thereof;
• tropical crop preferably selected from banana, cocoa, mango, pineapple, coffee, cassava, coconut, papaya, passion fruit, guava, mangosteen, oil palm fruit, and a combinations thereof;
• forage crops, preferably selected from silage corn, Brachiaria, bermudagrass, festuca, Lolium, Poa, alfalfa, Trifolium spp., Vicia spp., forage pea, Sorghum silage, oats, millet, silage root crops, feed cabbage, clovers, rye, ryegrass, and combinations thereof;
• ornamental plants preferably selected from Roses, Ornamental bulb plants, Geranium, Begonia, Petunia, Spathiphyllum, Marantha, Chrysanthemum, Tulips, Sterlitzia, passionflower, hydrangea, orchids, spider plant, snake plants, lilac, marigold, rosemary, wisteria, aromatic plants, and combinations thereof.

15. The extract or the formulation for use according, to anyone of claims 12-14, wherein the agronomic performance or trait is selected from:
• abiotic stress resistance and/or tolerance, preferably low or high temperature, deficient or excessive water, high salinity, heavy metals, and ultraviolet radiation; and/or
• nutrient use efficiency (NUE) and/or nutrient uptake, preferably said nutrient being selected from macro and/or meso/micro-nutrients, more preferably said macronutrients are selected from: nitrogen, phosphorus, potassium; and meso/micro-nutrients selected from: copper, sulphur, calcium, magnesium, iron, manganese, zinc, boron, and combinations thereof; and/or
• root growth and development and/or root biomass/weight and/or root system architecture; and/or
• shoot/canopy and/or root growth and development, biomass/weight; shoot greening, and/or
• yield potential and/or productivity of plants/crops; and/or
• plant fresh biomass and/or height and/or nutrient content and/or germination; and/or
• grain and/or fruit quality and quantity, number of pods, grain per pods, kernel line, grain weight, number of ears, flowering time, homogeneous budbreak/flowering, grain protein content, oil content, gluten strength, kernel size and vitreousness, fruit size, fruit setting, fruit color, fruit ripening, sugar content, improving flowering, pollination, and combinations thereof.
